# EUROPEAN PATENT APPLICATION

(11) **EP 4 603 505 A1**
(43) Date of publication of application: **20.08.2025**
(21) Application number: 23876784.2
(22) Date of filing: 12.10.2023
(51) Int. Cl.: C07K 14/015, C07K 14/005, C12N 15/864, C12N 15/866

(54) **METHOD FOR MODIFYING CAPSID PROTEIN CODING GENE OF ADENO-ASSOCIATED VIRUS**

(30) Priority: 13.10.2022 CN 202211254858
(71) Applicant: Kanglin Biotech (Hangzhou) Co., Ltd., Hangzhou, Zhejiang 310018 (CN)
(72) Inventor: WU, Haoquan, Hangzhou, Zhejiang 310018 (CN); SU, Lingling, Hangzhou, Zhejiang 310018 (CN); HAN, Lu, Hangzhou, Zhejiang 310018 (CN); MA, Yuxia, Hangzhou, Zhejiang 310018 (CN); ZHOU, Qunwei, Hangzhou, Zhejiang 310018 (CN)
(74) Representative: Maiwald GmbH
(86) International application number: PCT/CN2023/124269
(87) International publication number: WO 2024/078584

(57) **Abstract**

Provided are a method for modifying a capsid protein coding sequence of a cap gene of an adeno-associated virus (AAV), and an AAV capsid protein coding sequence obtained via the method. Additionally provided is the use of a modified AAV capsid protein coding sequence for expressing recombinant AAV and proteins of interest.

## Description

The present application claims priority of the prior Chinese application No. 202211254858.1 filed on October 13, 2022, the entire contents of which are incorporated herein by reference.

### TECHNICAL FIELD

The present disclosure relates to the field of recombinant vectors, and specifically to methods for modifying the capsid protein coding gene (cap gene) of adeno-associated virus (AAV).

### BACKGROUND

Adeno-associated virus (AAV) is classified under the Parvoviridae family. The virus particles of AAV are small, non-enveloped, and the capsid has an icosahedral structure. AAV is considered one of the most promising gene therapy candidate vectors currently, mainly because of its non-pathogenicity to humans and its ability to provide a long-term gene expression without inducing significant immune response.

The AAV capsid encapsulating the genome consists of a total of 60 subunits, including three different viral capsid proteins VP1, VP2, and VP3, whose gene sequences differ only at the N-terminus. The VP3 sequence is common among all three splice variants. VP2 and VP1 have relatively longer N-terminal sequences. The unique region of VP1 contains a phospholipase A2 domain, which plays an important role in the infectivity of the virus. All three types of VP proteins play key and specific roles in cell transduction, however, determining their precise stoichiometry remains challenging. In the wild-type capsid, the ratio of VP1, VP2 and VP3 is approximately 1:1:10. Since the assembly of the three capsid proteins is random, the ratio of VP1/VP2/VP3 in the recombinant AAV capsid depends mainly on their relative expression levels.

The classic method for producing recombinant AAV is to use plasmids to transiently transfect mammalian cells such as 293 cells or Hela cells, and then the recombinant AAV with a capsid structure similar to that of the wild type can be packaged. However, the culture properties of mammalian cells, such as adherent culture or the requirement for serum addition, result in many difficulties in scaling up. Accordingly, an increasing number of researchers are now using insect baculovirus systems for AAV packaging. However, the mechanism of producing recombinant AAV by infecting insect cells with recombinant baculovirus is very different from the method of transiently transfecting mammalian cells with plasmids. The natural hosts of wild-type AAV are mainly cells of mammals including primates. Some promoter sequences in the capsid protein coding sequence of AAV are very inefficient in insect cells. Therefore, in insect cells, it is usually difficult to achieve an efficient and proportional expression of three capsid proteins through a single gene sequence.

It can reduce the injection dose required to achieve the same therapeutic effect, thereby ensuring better safety, and reducing the cost and high-titer pressure of the production of recombinant viral vectors. Therefore, it is important to optimize the expression amount of the VP1 protein in cells.

### SUMMARY

In a first aspect, the present disclosure provides a method for modifying the capsid protein coding sequence of the capsid protein coding gene (cap gene) of an adeno-associated virus (AAV), wherein the cap gene encodes capsid proteins VP1, VP2 and VP3 through one coding sequence with different start codons, the method comprising:
(1) using a weak start codon for the coding sequences of the VP1 and VP2 and a strong start codon for the coding sequence of the VP3; and
(2) performing codon optimization for one or more codons in the coding sequence segment between the start codon of the coding sequence of the VP1 and the start codon of the coding sequence of the VP2 (VP1-specific segment), thereby reducing the initial translation strength of the codon.

The VP1-specific segment in the cap gene coding sequence is well known to those skilled in the art and can be determined by conventional methods. For example, in the capsid protein coding sequence of the AAV6 serotype cap gene shown in SEQ ID NO:2, the VP1 coding sequence refers to nucleotides at position 1 to position 411; in the capsid protein coding sequence of the AAV8 serotype cap gene shown in SEQ ID NO:4, the VP1-specific segment refers to nucleotides at position 1 to position 411.

In some embodiments, the start codon ACG is used for the coding sequence of VP1.

In some embodiments, the start codon ACG is used for the coding sequence of VP2.

In some embodiments, the start codon ATG is used for the coding sequence of VP3.

In some embodiments, in addition to the VP1-specific segment, one or more codons in additional coding sequence segment in the capsid protein coding sequence are codon-optimized to reduce the initial translation strength of the codon, and the additional coding sequence segment is selected from:
(1) a coding sequence segment between the start codon of the coding sequence of the VP2 and the start codon of the coding sequence of the VP3;
(2) a coding sequence segment downstream of the start codon of the coding sequence of the VP3; and
(3) a combination of (1) and (2).

The coding sequence segment between the start codon of the coding sequence of VP2 and the start codon of the coding sequence of VP3 in the cap gene coding sequence is well known to those skilled in the art and can be determined by conventional methods. For example, in the capsid protein coding sequence of the AAV6 serotype cap gene shown in SEQ ID NO: 2, the coding sequence segment between the start codon of the coding sequence of VP2 and the start codon of the coding sequence of VP3 refers to nucleotides at position 412 to position 609; in the capsid protein coding sequence of the AAV8 serotype cap gene shown in SEQ ID NO: 4, the coding sequence segment between the start codon of the coding sequence of VP2 and the start codon of the coding sequence of VP3 refers to nucleotides at position 412 to position 606.

The coding sequence segment downstream of the start codon of the VP3 coding sequence in the cap gene coding sequence is well known to those skilled in the art and can be determined by conventional methods. For example, in the capsid protein coding sequence of the AAV6 serotype cap gene shown in SEQ ID NO:2, the coding sequence segment downstream of the start codon of the VP3 coding sequence refers to nucleotides at position 610 to position 2214; in the capsid protein coding sequence of the AAV8 serotype cap gene shown in SEQ ID NO:4, the coding sequence segment downstream of the start codon of the VP3 coding sequence refers to nucleotides at position 607 to position 2208.

In some embodiments, all segments in the cap gene coding sequence are codon-optimized to reduce the initial translation strength of the codons in the segment.

In some embodiments of the above method, the codon optimization comprises one or more selected from the group consisting of:
(1) mutating the codon GAT coding aspartic acid (Asp) to GAC, preferably mutating all codons GAT in the segment to codons GAC;
(2) mutating the codon CTT coding leucine (Leu) to CTA, preferably mutating all codons CTT in the segment to codons CTA;
(3) mutating the codon TTG coding leucine (Leu) to CTC, preferably mutating all codons TTG in the segment to codons CTC;
(4) mutating the codon ATT coding isoleucine (Ile) to ATC, preferably mutating all codons ATT in the segment to codons ATC;
(5) mutating the codon CCA coding proline (Pro) to CCC, preferably mutating all codons CCA in the segment to codons CCC;
(6) mutating the codon CTG coding leucine (Leu) to CTC, preferably mutating all codons CTG in the segment to codons CTC;
(7) mutating the codon GTG coding valine (Val) to GTC, preferably mutating all codons GTG in the segment to codons GTC;
(8) mutating the codon GAA coding glutamic acid (Glu) to GAG, preferably mutating all codons GAA in the segment to codons GAG; and
(9) mutating the codon ACG coding threonine (Thr) to ACC, preferably mutating all codons ACG in the segment to codons ACC.

In some embodiments, the codon optimization comprises at least 2, at least 3, at least 4, at least 5, at least 6, at least 7, all 8 or all 9 of (1) to (9).

In some embodiments, the codon optimization comprises:
(1) mutating the codon TTG coding leucine (Leu) to CTC, preferably mutating all codons TTG in the segment to codons CTC;
(2) mutating the codon CTG coding leucine (Leu) to CTC, preferably mutating all codons CTG in the segment to codons CTC;
(3) mutating the codon GTG coding valine (Val) to GTC, preferably mutating all codons GTG in the segment to codons GTC; and
(4) mutating the codon ACG coding threonine (Thr) to ACC, preferably mutating all codons ACG in the segment to codons ACC.

In some embodiments, the AAV is of a serotype selected from the group consisting of: AAV1, AAV2, AAV3, AAV4, AAV5, AAV6, AAV7, AAV8 and AAV9. In some preferred embodiments, the AAV is of AAV6 or AAV8 serotype.

The wild-type cap gene coding sequences of various AAV serotypes can be conventionally obtained by those skilled in the art. For example, the wild-type cap gene of AAV1 serotype may have a nucleotide sequence as shown in SEQ ID NO: 13; the wild-type cap gene of AAV2 serotype may have a nucleotide sequence as shown in SEQ ID NO: 14; the wild-type cap gene of AAV3 serotype may have a nucleotide sequence as shown in SEQ ID NO: 15; the wild-type cap gene of AAV4 serotype may have a nucleotide sequence as shown in SEQ ID NO: 16; the wild-type cap gene of AAV5 serotype may have a nucleotide sequence as shown in SEQ ID NO: 17; the wild-type cap gene of AAV6 serotype may have a nucleotide sequence as shown in SEQ ID NO: 18; the wild-type cap gene of AAV7 serotype may have a nucleotide sequence as shown in SEQ ID NO: 19; the wild-type cap gene of AAV8 serotype may have a nucleotide sequence as shown in SEQ ID NO:20; the wild-type cap gene of AAV9 serotype may have a nucleotide sequence as shown in SEQ ID NO:21.

In some embodiments, the AAV is of AAV6 serotype, and the codon optimization comprises the following codon optimization of the codons in the VP1-specific segment:
(1) mutating the codon GAT coding aspartic acid (Asp) to GAC, preferably mutating all codons GAT in the segment to codons GAC;
(2) mutating the codon ATT coding isoleucine (Ile) to ATC, preferably mutating all codons ATT in the segment to codons ATC;
(3) mutating the codon TTG coding leucine (Leu) to CTC, preferably mutating all codons TTG in the segment to codons CTC;
(4) mutating the codon CTG coding leucine (Leu) to CTC, preferably mutating all codons CTG in the segment to codons CTC;
(5) mutating the codon GTG coding valine (Val) to GTC, preferably mutating all codons GTG in the segment to codons GTC;
(6) mutating the codon ACG coding threonine (Thr) to ACC, preferably mutating all codons ACG in the segment to codons ACC.

In some embodiments, the AAV is of AAV8 serotype, and the codon optimization comprises the following codon optimization of the codons in the VP1-specific segment:
(1) mutating the codon GAT coding aspartic acid (Asp) to GAC, preferably mutating all codons GAT in the segment to codons GAC;
(2) mutating the codon CTT coding leucine (Leu) to CTA, preferably mutating all codons CTT in the segment to codons CTA;
(3) mutating the codon CCA coding proline (Pro) to CCC, preferably mutating all codons CCA in the segment to codons CCC;
(4) mutating the codon CTG coding leucine (Leu) to CTC, preferably mutating all codons CTG in the segment to codons CTC;
(5) mutating the codon GTG coding valine (Val) to GTC, preferably mutating all codons GTG in the segment to codons GTC;
(6) mutating the codon GAA coding glutamic acid (Glu) to GAG, preferably mutating all codons GAA in the segment to codons GAG;
(7) mutating the codon ACG coding threonine (Thr) to ACC, preferably mutating all codons ACG in the segment to codons ACC.

In a second aspect, the present disclosure relates to a capsid protein coding sequence of the cap gene of AAV obtained by the method of the first aspect of the present disclosure.

In a third aspect, the present disclosure relates to a capsid protein coding sequence of the cap gene of a recombinant AAV, the capsid protein coding sequence has a nucleotide sequence as shown in SEQ ID NO:3 or SEQ ID NO:5, or a variant nucleotide sequence having at least 80%, at least 85%, at least 90%, at least 95%, at least 98%, or at least 99% sequence identity with the sequence shown in SEQ ID NO:3 or SEQ ID NO:5.

In a fourth aspect, the present disclosure relates to a capsid protein coding sequence of the cap gene of a recombinant AAV, wherein the cap gene encodes capsid proteins VP1, VP2 and VP3 through one coding sequence with different start codons, wherein:
(1) the coding sequences of the VP1 and VP2 have weak start codons and the coding sequence of the VP3 has a strong start codon;
(2) one or more codons in the coding sequence segment between the start codon of the coding sequence of the VP1 and the start codon of the coding sequence of the VP2 (VP1-specific segment) are codon-optimized, thereby reducing the initial translation strength of the one or more codons.

In some embodiments, the coding sequence of VP1 has a start codon ACG.

In some embodiments, the coding sequence of VP2 has a start codon ACG.

In some embodiments, the coding sequence of VP3 has a start codon ATG.

In some embodiments, the coding sequences of VP1 and VP2 have start codon ACG, and the coding sequence of VP3 has a start codon ATG.

In some embodiments, in addition to the VP1-specific segment, one or more codons in additional coding sequence segment in the capsid protein coding sequence are also codon-optimized to reduce the initial translation strength of the codon, and said additional coding sequence segment is selected from:
(1) a coding sequence segment between the start codon of the coding sequence of the VP2 and the start codon of the coding sequence of the VP3;
(2) a coding sequence segment downstream of the start codon of the coding sequence of the VP3; and
(3) a combination of (1) and (2).

In some embodiments of the above-mentioned capsid protein coding sequence of the cap gene of a recombinant AAV, the codon optimization comprises one or more selected from the group consisting of:
(1) mutating the codon GAT coding aspartic acid (Asp) to GAC, preferably mutating all codons GAT in the segment to codons GAC;
(2) mutating the codon CTT coding leucine (Leu) to CTA, preferably mutating all codons CTT in the segment to codons CTA;
(3) mutating the codon TTG coding leucine (Leu) to CTC, preferably mutating all codons TTG in the segment to codons CTC;
(4) mutating the codon ATT coding isoleucine (Ile) to ATC, preferably mutating all codons ATT in the segment to codons ATC;
(5) mutating the codon CCA coding proline (Pro) to CCC, preferably mutating all codons CCA in the segment to codons CCC;
(6) mutating the codon CTG coding leucine (Leu) to CTC, preferably mutating all codons CTG in the segment to codons CTC;
(7) mutating the codon GTG coding valine (Val) to GTC, preferably mutating all codons GTG in the segment to codons GTC;
(8) mutating the codon GAA coding glutamic acid (Glu) to GAG, preferably mutating all codons GAA in the segment to codons GAG; and
(9) mutating the codon ACG coding threonine (Thr) to ACC, preferably mutating all codons ACG in the segment to codons ACC.

In some embodiments, the codon optimization comprises at least 2, at least 3, at least 4, at least 5, at least 6, at least 7, all 8 or all 9 of (1) to (9).

In some embodiments, the codon optimization comprises:
(1) mutating the codon TTG coding leucine (Leu) to CTC, preferably mutating all codons TTG in the segment to codons CTC;
(2) mutating the codon CTG coding leucine (Leu) to CTC, preferably mutating all codons CTG in the segment to codons CTC;
(3) mutating the codon GTG coding valine (Val) to GTC, preferably mutating all codons GTG in the segment to codons GTC; and
(4) mutating the codon ACG coding threonine (Thr) to ACC, preferably mutating all codons ACG in the segment to codons ACC.

In some embodiments, the AAV is of a serotype selected from the group consisting of: AAV1, AAV2, AAV3, AAV4, AAV5, AAV6, AAV7, AAV8 and AAV9, preferably the AAV is of the AAV6 or AAV8 serotype.

In some embodiments, the AAV is of AAV6 serotype, and the codon optimization comprises the following codon optimization of the codons in the VP1-specific segment:
(1) mutating the codon GAT coding aspartic acid (Asp) to GAC, preferably mutating all codons GAT in the segment to codons GAC;
(2) mutating the codon ATT coding isoleucine (Ile) to ATC, preferably mutating all codons ATT in the segment to codons ATC;
(3) mutating the codon TTG coding leucine (Leu) to CTC, preferably mutating all codons TTG in the segment to codons CTC;
(4) mutating the codon CTG coding leucine (Leu) to CTC, preferably mutating all codons CTG in the segment to codons CTC;
(5) mutating the codon GTG coding valine (Val) to GTC, preferably mutating all codons GTG in the segment to codons GTC;
(6) mutating the codon ACG coding threonine (Thr) to ACC, preferably mutating all codons ACG in the segment to codons ACC.

In some embodiments, the AAV is of AAV8 serotype, and the codon optimization comprises the following codon optimization of the codons in the VP1-specific segment:
(1) mutating the codon GAT coding aspartic acid (Asp) to GAC, preferably mutating all codons GAT in the segment to codons GAC;
(2) mutating the codon CTT coding leucine (Leu) to CTA, preferably mutating all codons CTT in the segment to codons CTA;
(3) mutating the codon CCA coding proline (Pro) to CCC, preferably mutating all codons CCA in the segment to codons CCC;
(4) mutating the codon CTG coding leucine (Leu) to CTC, preferably mutating all codons CTG in the segment to codons CTC;
(5) mutating the codon GTG coding valine (Val) to GTC, preferably mutating all codons GTG in the segment to codons GTC;
(6) mutating the codon GAA coding glutamic acid (Glu) to GAG, preferably mutating all codons GAA in the segment to codons GAG;
(7) mutating the codon ACG coding threonine (Thr) to ACC, preferably mutating all codons ACG in the segment to codons ACC.

In some embodiments, the capsid protein coding sequence of the cap gene of a recombinant AAV has a nucleotide sequence as shown in SEQ ID NO:3 or SEQ ID NO:5, or a variant nucleotide sequence having at least 80% sequence identity, at least 85% sequence identity, at least 90% sequence identity, at least 95% sequence identity, at least 98% sequence identity, or at least 99% sequence identity with the sequence shown in SEQ ID NO:3 or SEQ ID NO:5.

In a fifth aspect, the present disclosure relates to a vector comprising the capsid protein coding sequence of the cap gene of a recombinant AAV according to the fourth aspect of the present disclosure.

In some embodiments, the vector is an expression vector.

In some embodiments, the vector is a viral vector, preferably an insect baculovirus vector.

In some embodiments, the vector is derived from a vector system selected from the group consisting of: Bac-to-Bac system, flashBac/BacMagic system, BaculoDirect system and BacPAK6/Bacologold system.

In some embodiments, the vector is derived from the Bac-to-Bac system. The Bac-to-Bac system vector comprises vector backbones such as pFastBacdual, pFastBac1, pFastBacHTA, pFastBacHTB or pFastBacHTC. In some preferred embodiments, the vector is derived from the pFastBacdual vector backbone.

In some embodiments, the vector further comprises the gene of replication-related protein (rep gene) of AAV. In some embodiments, the rep gene is selected from a rep gene of AAV1, AAV2, AAV3, AAV4, AAV5, AAV6, AAV7, AAV8 and AAV9. In a preferred embodiment, the rep gene is derived from AAV2 serotype.

In some embodiments, the vector comprises an expression regulatory sequence operably linked to the capsid protein coding sequence of the cap gene and/or to the rep gene of a recombinant AAV. In some embodiments, the expression regulatory sequence comprises one or more promoters and/or one or more enhancers.

In some embodiments, the promoter is selected from one or more of pH, Gp64, p6.9 and p10 promoters. In some preferred embodiments, the promoter comprises a combination of hr1 and p6.9 promoter, and p10 promoter (hrlp6.9p10), a combination of pH promoter and Acie01 (pH-Acie01), or a single pH promoter.

In some embodiments, the enhancer is selected from one or more of hr1, hr2, hr3, hr4, and hr5 enhancers.

In some embodiments, the vector further comprises a sequence coding a protein of interest. In some embodiments, the protein of interest is selected from coagulation factors such as FIX and FVIII, antibodies or fragments thereof such as scFv fragments, and dopamine synthesis proteins, etc.

In a sixth aspect, the present disclosure relates to a cell comprising the vector of the fifth aspect of the present disclosure. In some embodiments, the cell is an insect cell.

In a seventh aspect, the present disclosure relates to a method for preparing a recombinant AAV, the method comprising:
(1) culturing the cell according to the sixth aspect of the present disclosure; and
(2) isolating the recombinant AAV from the cell and/or the culture supernatant.

In some embodiments, when expressed in the cell, the expression amount of VP1, VP2 and VP3 is in a ratio of about 1:1:10.

In an eighth aspect, the present disclosure provides a composition, the composition comprising the capsid protein coding gene of a recombinant AAV according to the second aspect or the third aspect of the present disclosure, the vector according to the fourth aspect of the present disclosure or the cell according to the fifth aspect of the present disclosure. In some embodiments, the composition further comprises a pharmaceutically acceptable carrier and/or excipient.

The present disclosure also relates to the following embodiments:
Embodiment 1. A method for modifying the capsid protein coding sequence of the capsid protein coding gene (cap gene) of an adeno-associated virus (AAV), wherein the cap gene encodes capsid proteins VP1, VP2 and VP3 through one coding sequence with different start codons, the method comprising:
   (1) using a weak start codon for the coding sequence of the VP1; and
   (2) performing codon optimization for one or more codons in the coding sequence segment between the start codon of the coding sequence of the VP1 and the start codon of the coding sequence of the VP2 (VP1-specific segment), thereby reducing the initial translation strength of the codon.
Embodiment 2. The method according to Embodiment 1, wherein:
   (1) using the start codon ACG for the coding sequence of the VP1; and/or
   (2) the codon optimization in the VP1-specific segment comprises: mutating all or part of the codons with stronger initiation function in the VP1-specific segment into synonymous codons except for the above codons.
Embodiment 3. A method for modifying the capsid protein coding sequence of the capsid protein coding gene (cap gene) of an adeno-associated virus (AAV), wherein the cap gene encodes capsid proteins VP1, VP2 and VP3 through one coding sequence with different start codons, the method comprising:
   (1) using a weak start codon for the coding sequences of the VP1 and VP2 and a strong start codon for the coding sequence of the VP3; and
   (2) performing codon optimization for one or more codons in the coding sequence segment between the start codon of the coding sequence of the VP1 and the start codon of the coding sequence of the VP2 (VP1-specific segment), thereby reducing the initial translation strength of the codon.
Embodiment 4. The method according to Embodiment 3, wherein the start codon ACG is used for the coding sequences of the VP1 and VP2, and the start codon ATG is used for the coding sequence of VP3.
Embodiment 5. The method according to any one of Embodiments 1 to 4, wherein in addition to the VP1-specific segment, one or more codons in additional coding sequence segment in the capsid protein coding sequence are codon-optimized to reduce the initial translation strength of the codon, and said additional coding sequence segment is selected from:
   (1) a coding sequence segment between the start codon of the coding sequence of the VP2 and the start codon of the coding sequence of the VP3;
   (2) a coding sequence segment downstream of the start codon of the coding sequence of the VP3; and
   (3) a combination of (1) and (2).
Embodiment 6. The method according to any one of Embodiments 1 to 5, wherein the codon optimization comprises one or more selected from the group consisting of:
   (1) mutating the codon GAT coding aspartic acid (Asp) to a synonymous codon except for GAT, preferably to GAC, preferably mutating all GAT codons in the segment;
   (2) mutating the codon CTT coding leucine (Leu) to a synonymous codon except for CTT, preferably to CTA, preferably mutating all CTT codons in the segment;
   (3) mutating the codon TTG coding leucine (Leu) to a synonymous codon except for TTG, preferably to CTC, preferably mutating all TTG codons in the segment;
   (4) mutating the codon ATT coding isoleucine (Ile) to a synonymous codon except for ATT, preferably to ATC, preferably mutating all ATT codons in the segment;
   (5) mutating the codon CCA coding proline (Pro) to a synonymous codon except for CCA, preferably to CCC, preferably mutating all CCA codons in the segment;
   (6) mutating the codon CTG coding leucine (Leu) to a synonymous codon except for CTG, preferably to CTC, preferably mutating all CTG codons in the segment;
   (7) mutating the codon GTG coding valine (Val) to a synonymous codon except for GTG, preferably to GTC, preferably mutating all GTG codons in the segment;
   (8) mutating the codon GAA coding glutamic acid (Glu) to a synonymous codon except for GAA, preferably to GAG, preferably mutating all GAA codons in the segment; and
   (9) mutating the codon ACG coding threonine (Thr) to a synonymous codon except for ACG, preferably to ACC, preferably mutating all ACG codons in the segment.
Embodiment 7. The method according to Embodiment 6, wherein the codon optimization comprises at least 2, at least 3, at least 4, at least 5, at least 6, at least 7, all 8 or all 9 of items (1) to (9).
Embodiment 8. The method according to Embodiment 6 or 7, wherein the codon optimization comprises one or more selected from the group consisting of:
   (1) mutating the codon TTG coding leucine (Leu) to a synonymous codon except for TTG, preferably to CTC, preferably mutating all TTG codons in the segment;
   (2) mutating the codon CTG coding leucine (Leu) to a synonymous codon except for CTG, preferably to CTC, preferably mutating all CTG codons in the segment;
   (3) mutating the codon GTG coding valine (Val) to a synonymous codon except for GTG, preferably to GTC, preferably mutating all GTG codons in the segment; and
   (4) mutating the codon ACG coding threonine (Thr) to a synonymous codon except for ACG, preferably to ACC, preferably mutating all ACG codons in the segment.
Embodiment 9. The method according to Embodiment 8, wherein the codon optimization comprises at least 2, at least 3 or all 4 of items (1) to (4).
Embodiment 10. The method according to any one of Embodiments 1 to 9, wherein the method further comprises: after the codon optimization, a trinucleotide ATG present in two adjacent codons in the capsid protein coding sequence is optimized, and the trinucleotide ATG is mutated to a non-ATG sequence without changing the amino acid encoded by the two adjacent codons.
Embodiment 11. The method according to any one of Embodiments 1 to 10, wherein the AAV is of a serotype selected from the group consisting of AAV1, AAV2, AAV3, AAV4, AAV5, AAV6, AAV7, AAV8 and AAV9, preferably the AAV is of a serotype of AAV6, AAV8 or AAV9.
Embodiment 12. The method according to Embodiment 11, wherein the AAV is of AAV6 serotype, and the codon optimization comprises the following codon optimization of the codons in the VP1-specific segment:
   (1) mutating the codon GAT coding aspartic acid (Asp) to a synonymous codon except for GAT, preferably to GAC, preferably mutating all GAT codons in the segment;
   (2) mutating the codon ATT coding isoleucine (Ile) to a synonymous codon except for ATT, preferably to ATC, preferably mutating all ATT codons in the segment;
   (3) mutating the codon TTG coding leucine (Leu) to a synonymous codon except for TTG, preferably to CTC, preferably mutating all TTG codons in the segment;
   (4) mutating the codon CTG coding leucine (Leu) to a synonymous codon except for CTG, preferably to CTC, preferably mutating all CTG codons in the segment;
   (5) mutating the codon GTG coding valine (Val) to a synonymous codon except for GTG, preferably to GTC, preferably mutating all GTG codons in the segment;
   (6) mutating the codon ACG coding threonine (Thr) to a synonymous codon except for ACG, preferably to ACC, preferably mutating all ACG codons in the segment.
Embodiment 13. The method according to Embodiment 11, wherein the AAV is of AAV8 serotype, and the codon optimization comprises the following codon optimization of the codons in the VP1-specific segment:
   (1) mutating the codon GAT coding aspartic acid (Asp) to a synonymous codon except for GAT, preferably to GAC, preferably mutating all GAT codons in the segment;
   (2) mutating the codon CTT coding leucine (Leu) to a synonymous codon except for CTT, preferably to CTA, preferably mutating all CTT codons in the segment;
   (3) mutating the codon CCA coding proline (Pro) to a synonymous codon except for CCA, preferably to CCC, preferably mutating all CCA codons in the segment;
   (4) mutating the codon CTG coding leucine (Leu) to a synonymous codon except for CTG, preferably to CTC, preferably mutating all CTG codons in the segment;
   (5) mutating the codon GTG coding valine (Val) to a synonymous codon except for GTG, preferably to GTC, preferably mutating all GTG codons in the segment;
   (6) mutating the codon GAA coding glutamic acid (Glu) to a synonymous codon except for GAA, preferably to GAG, preferably mutating all GAA codons in the segment;
   (7) mutating the codon ACG coding threonine (Thr) to a synonymous codon except for ACG, preferably to ACC, preferably mutating all ACG codons in the segment.
Embodiment 14. The method according to Embodiment 11, wherein the AAV is of AAV9 serotype, and the codon optimization comprises the following codon optimization of the codons in the VP1-specific segment:
   (1) mutating the codon TTG coding leucine (Leu) to a synonymous codon except for TTG, preferably to TTA, CTA, CTC or CTT, preferably mutating all TTG codons in the segment;
   (2) mutating the codon CTG coding leucine (Leu) to a synonymous codon except for CTG, preferably to CTC, preferably mutating all CTG codons in the segment;
   (3) mutating the codon GTG coding valine (Val) to a synonymous codon except for GTG, preferably to GTC, preferably mutating all GTG codons in the segment;
   (4) mutating the codon ACG coding threonine (Thr) to a synonymous codon except for ACG, preferably to ACC, preferably mutating all ACG codons in the segment.
Embodiment 15. The method according to Embodiment 14, wherein the method further comprises:
   after the codon optimization, a trinucleotide ATG present in two adjacent codons in the capsid protein coding sequence is optimized, and the trinucleotide ATG is mutated to a non-ATG sequence without changing the amino acid encoded by the two adjacent codons.
   Embodiment 16. The capsid protein coding sequence of the cap gene of an AAV obtained by the method of any one of Embodiments 1 to 15.
Embodiment 17. A capsid protein coding sequence of the cap gene of a recombinant AAV, which has a nucleotide sequence as shown in SEQ **ID** NO:3 or SEQ **ID** NO:5, or a variant nucleotide sequence having at least 80%, at least 85%, at least 90%, at least 95%, at least 98%, or at least 99% sequence identity with the sequence shown in SEQ **ID** NO:3 or SEQ **ID** NO:5.
Embodiment 18. A capsid protein coding sequence of the cap gene of a recombinant AAV, wherein the VP1 promoter and the VP1-specific segment in the coding sequence have a nucleotide sequence as shown in any one of SEQ ID NOs:23-26, or a variant nucleotide sequence having at least 80%, at least 85%, at least 90%, at least 95%, at least 98%, or at least 99% sequence identity with the sequence shown in any one of SEQ ID NOs:23-26.
Embodiment 19. A capsid protein coding sequence of the cap gene of a recombinant AAV, wherein the cap gene encodes capsid proteins VP1, VP2 and VP3 through one coding sequence with different start codons, wherein:
   (1) the coding sequence of the VP1 has a weak start codon;
   (2) one or more codons in the coding sequence segment between the start codon of the coding sequence of the VP1 and the start codon of the coding sequence of the VP2 (VP1-specific segment) are codon-optimized, thereby reducing the initial translation strength of the codon.
Embodiment 20. The capsid protein coding sequence according to Embodiment 19, wherein:
   (1) the coding sequence of the VP1 has a start codon ACG; and/or
   (2) codon optimization in the VP1-specific segment comprises: mutating all or part of the codons with stronger initiation function in the VP1-specific segment into synonymous codons except for the above codons.
Embodiment 21. A capsid protein coding sequence of the cap gene of a recombinant AAV, wherein the cap gene encodes capsid proteins VP1, VP2 and VP3 through one coding sequence with different start codons, wherein:
   (1) the coding sequences of the VP1 and VP2 have weak start codons and the coding sequence of the VP3 has a strong start codon;
   (2) one or more codons in the coding sequence segment between the start codon of the coding sequence of the VP1 and the start codon of the coding sequence of the VP2 (VP1-specific segment) are codon-optimized, thereby reducing the initial translation strength of the codon.
Embodiment 22. The capsid protein coding sequence of the cap gene of a recombinant AAV according to Embodiment 21, wherein the coding sequences of the VP1 and VP2 have a start codon ACG, and the coding sequence of the VP3 has a start codon ATG.
Embodiment 23. The capsid protein coding sequence of the cap gene of a recombinant AAV according to any one of Embodiments 19 to 22, wherein in addition to the VP1-specific segment, one or more codons in additional coding sequence segment in the capsid protein coding sequence are also codon-optimized to reduce the initial translation strength of the codon, and said additional coding sequence segment is selected from:
   (1) a coding sequence segment between the start codon of the coding sequence of the VP2 and the start codon of the coding sequence of the VP3;
   (2) a coding sequence segment downstream of the start codon of the coding sequence of the VP3; and
   (3) a combination of (1) and (2).
Embodiment 24. The capsid protein coding sequence of the cap gene of a recombinant AAV according to any one of Embodiments 19 to 23, wherein the codon optimization comprises one or more selected from the group consisting of:
   (1) mutating the codon GAT coding aspartic acid (Asp) to a synonymous codon except for GAT, preferably to GAC, preferably mutating all GAT codons in the segment;
   (2) mutating the codon CTT coding leucine (Leu) to a synonymous codon except for CTT, preferably to CTA, preferably mutating all CTT codons in the segment;
   (3) mutating the codon TTG coding leucine (Leu) to a synonymous codon except for TTG, preferably to CTC, preferably mutating all TTG codons in the segment;
   (4) mutating the codon ATT coding isoleucine (Ile) to a synonymous codon except for ATT, preferably to ATC, preferably mutating all ATT codons in the segment;
   (5) mutating the codon CCA coding proline (Pro) to a synonymous codon except for CCA, preferably to CCC, preferably mutating all CCA codons in the segment;
   (6) mutating the codon CTG coding leucine (Leu) to a synonymous codon except for CTG, preferably to CTC, preferably mutating all CTG codons in the segment;
   (7) mutating the codon GTG coding valine (Val) to a synonymous codon except for GTG, preferably to GTC, preferably mutating all GTG codons in the segment;
   (8) mutating the codon GAA coding glutamic acid (Glu) to a synonymous codon except for GAA, preferably to GAG, preferably mutating all GAA codons in the segment; and
   (9) mutating the codon ACG coding threonine (Thr) to a synonymous codon except for ACG, preferably to ACC, preferably mutating all ACG codons in the segment.
Embodiment 25. The capsid protein coding sequence of the cap gene of a recombinant AAV according to Embodiment 24, wherein the codon optimization comprises at least 2, at least 3, at least 4, at least 5, at least 6, at least 7, all 8 or all 9 of items (1) to (9).
Embodiment 26. The capsid protein coding sequence of the cap gene of a recombinant AAV according to Embodiment 24 or 25, wherein the codon optimization comprises one or more selected from the group consisting of:
   (1) mutating the codon TTG coding leucine (Leu) to a synonymous codon except for TTG, preferably to CTC, preferably mutating all TTG codons in the segment;
   (2) mutating the codon CTG coding leucine (Leu) to a synonymous codon except for CTG, preferably to CTC, preferably mutating all CTG codons in the segment;
   (3) mutating the codon GTG coding valine (Val) to a synonymous codon except for GTG, preferably to GTC, preferably mutating all GTG codons in the segment; and
   (4) mutating the codon ACG coding threonine (Thr) to a synonymous codon except for ACG, preferably to ACC, preferably mutating all ACG codons in the segment.
Embodiment 27. The capsid protein coding sequence according to Embodiment 26, wherein the codon optimization comprises at least 2, at least 3 or all 4 of items (1) to (4).
Embodiment 28. The capsid protein coding sequence according to any one of Embodiments 19 to 27, wherein a trinucleotide ATG present in two adjacent codons in the capsid protein coding sequence is optimized, and the trinucleotide ATG is mutated into a non-ATG sequence without changing the amino acid encoded by the two adjacent codons.
Embodiment 29. The capsid protein coding sequence of the cap gene of a recombinant AAV according to any one of Embodiments 19 to 28, wherein the AAV is of a serotype selected from the group consisting of: AAV1, AAV2, AAV3, AAV4, AAV5, AAV6, AAV7, AAV8 and AAV9, preferably the AAV is a serotype of AAV6, AAV8 or AAV9.
Embodiment 30. The capsid protein coding sequence according to Embodiment 29, wherein the AAV is of AAV6 serotype, and the codon optimization comprises the following codon optimization of the codons in the VP1-specific segment:
   (1) mutating the codon GAT coding aspartic acid (Asp) to a synonymous codon except for GAT, preferably to GAC, preferably mutating all GAT codons in the segment;
   (2) mutating the codon ATT coding isoleucine (Ile) to a synonymous codon except for ATT, preferably to ATC, preferably mutating all ATT codons in the segment;
   (3) mutating the codon TTG coding leucine (Leu) to a synonymous codon except for TTG, preferably to CTC, preferably mutating all TTG codons in the segment;
   (4) mutating the codon CTG coding leucine (Leu) to a synonymous codon except for CTG, preferably to CTC, preferably mutating all CTG codons in the segment;
   (5) mutating the codon GTG coding valine (Val) to a synonymous codon except for GTG, preferably to GTC, preferably mutating all GTG codons in the segment;
   (6) mutating the codon ACG coding threonine (Thr) to a synonymous codon except for ACG, preferably to ACC, preferably mutating all ACG codons in the segment.
Embodiment 31. The capsid protein coding sequence according to Embodiment 29, wherein the AAV is of AAV8 serotype, and the codon optimization comprises the following codon optimization of the codons in the VP1-specific segment:
   (1) mutating the codon GAT coding aspartic acid (Asp) to a synonymous codon except for GAT, preferably to GAC, preferably mutating all GAT codons in the segment;
   (2) mutating the codon CTT coding leucine (Leu) to a synonymous codon except for CTT, preferably to CTA, preferably mutating all the CTT codons in the segment;
   (3) mutating the codon CCA coding proline (Pro) to a synonymous codon except for CCA, preferably to CCC, preferably mutating all CCA codons in the segment;
   (4) mutating the codon CTG coding leucine (Leu) to a synonymous codon except for CTG, preferably to CTC, preferably mutating all CTG codons in the segment;
   (5) mutating the codon GTG coding valine (Val) to a synonymous codon except for GTG, preferably to GTC, preferably mutating all GTG codons in the segment;
   (6) mutating the codon GAA coding glutamic acid (Glu) to a synonymous codon except for GAA, preferably to GAG, preferably mutating all GAA codons in the segment;
   (7) mutating the codon ACG coding threonine (Thr) to a synonymous codon except for ACG, preferably to ACC, preferably mutating all ACG codons in the segment.
Embodiment 32. The capsid protein coding sequence according to Embodiment 29, wherein the AAV is of AAV9 serotype, and the codon optimization comprises the following codon optimization of the codons in the VP1-specific segment:
   (1) mutating the codon TTG coding leucine (Leu) to a synonymous codon except for TTG, preferably to TTA, CTA, CTC or CTT, preferably mutating all TTG codons in the segment;
   (2) mutating the codon CTG coding leucine (Leu) to a synonymous codon except for CTG, preferably to CTC, preferably mutating all CTG codons in the segment;
   (3) mutating the codon GTG coding valine (Val) to a synonymous codon except for GTG, preferably to GTC, preferably mutating all GTG codons in the segment;
   (4) mutating the codon ACG coding threonine (Thr) to a synonymous codon except for ACG, preferably to ACC, preferably mutating all ACG codons in the segment.
Embodiment 33. The capsid protein coding sequence according to Embodiment 32, wherein a trinucleotide ATG present in two adjacent codons in the capsid protein coding sequence is optimized, and the trinucleotide ATG is mutated into a non-ATG sequence without changing the amino acid encoded by the two adjacent codons.
Embodiment 34. The capsid protein coding sequence of the cap gene of a recombinant AAV according to Embodiment 19, which has a nucleotide sequence as shown in SEQ **ID** NO:3 or SEQ **ID** NO:5, or a variant nucleotide sequence having at least 80%, at least 85%, at least 90%, at least 95%, at least 98%, or at least 99% sequence identity with the sequence shown in SEQ ID NO:3 or SEQ ID NO:5.
Embodiment 35. The capsid protein coding sequence of the cap gene of a recombinant AAV according to Embodiment 19, wherein the VP1 promoter and the VP1-specific segment in the coding sequence have a nucleotide sequence as shown in any one of SEQ ID NOs:23-26, or a variant nucleotide sequence having at least 80%, at least 85%, at least 90%, at least 95%, at least 98%, or at least 99% sequence identity with the sequence shown in any one of SEQ ID NOs:23-26.
Embodiment 36. A vector comprising the capsid protein coding sequence of the cap gene of a recombinant AAV according to any one of Embodiments 19 to 35, preferably the vector is an expression vector.
Embodiment 37. The vector according to Embodiment 36, wherein the vector is a viral vector, preferably an insect baculovirus vector.
Embodiment 38. The vector according to Embodiment 37, wherein the vector is derived from a vector system selected from: Bac-to-Bac vector system, flashBac/BacMagic vector system, BaculoDirect vector system or BacPAK6/Bacologold vector system.
Embodiment 39. The vector according to any one of Embodiments 36 to 38, wherein the vector further comprises a replication-associated protein gene (rep gene) of AAV, preferably the rep gene is derived from AAV2 serotype.
Embodiment 40. The vector according to any one of Embodiments 36 to 39, wherein the vector further comprises a nucleotide sequence coding a protein of interest.
Embodiment 41. A cell, wherein the cell comprises the vector according to any one of Embodiments 36 to 40, preferably the cell is an insect cell.
Embodiment 42. A method for preparing a recombinant AAV, the method comprising:
   (1) culturing the cell according to Embodiment 41; and
   (2) isolating the recombinant AAV from the cell and/or the culture supernatant.
Embodiment 43. The method according to Embodiment 42, wherein when expressed in the cell, the expression amount of VP1, VP2 and VP3 is in a ratio of about 1:1:10.

### DESCRIPTION OF THE DRAWINGS

Fig. 1 shows the strategy of using start codons with different strengths for the VP1, VP2 and VP3 coding sequences in the capsid protein coding gene of AAV.
Fig. 2 shows a previous optimization strategy of adding an intron sequence comprising an insect cell promoter to the capsid protein coding gene of AAV.
Fig. 3 shows a strategy for codon optimization of the capsid protein coding gene of AAV.
Fig. 4 shows a recombinant sequence comprising ITR-CMV-EGFP.
Fig. 5 shows a map of the recombinant baculovirus shuttle vector pFBd-ITR-EGFP.
Fig. 6 shows a recombinant sequence comprising an optimized Cap gene and other elements.
Fig. 7 shows a map of the recombinant baculovirus shuttle vector pFBd-IE-hr1Cap-Rep.
Fig. 8 shows the results of the expression ratios of the three capsid proteins before and after optimization detected by gel electrophoresis and Coomassie Brilliant Blue staining.
Fig. 9 shows the results of the expression ratios of capsid proteins of five AAV9 serotypes before and after optimization detected by gel electrophoresis and Coomassie Brilliant Blue staining.

### DETAILED DESCRIPTION

Unless otherwise defined, all technical and scientific terms used herein have the same meaning as commonly understood by one of ordinary skilled person in the art to which the present disclosure belongs.

In order to make it easier to understand the present disclosure, certain terms are first defined below. Additional definitions of the following terms and other terms are set forth throughout the specification.

As used in the specification and the appended claims, the singular forms "a", "an" and "the" include plural referents unless otherwise expressly stated.

The terms "comprising", "having", "including" and "containing" used herein should be interpreted as open-ended terms (i.e., meaning "including but not limited to").

All publications and other references mentioned herein are incorporated herein by reference in their entirety. Although many references are cited herein, the citation does not constitute an admission that any of these publications form part of the common general knowledge in the art.

The phrase "AAV" or "adeno-associated virus" refers to the Dependoparvovirus of the Parvoviridae genus of viruses. AAV may be derived from a naturally occurring "wild-type" virus, a rAAV genome packaged into a capsid derived from a capsid protein encoded by a naturally occurring cap gene, and/or a rAAV genome packaged into a capsid derived from a capsid protein encoded by a non-natural capsid cap gene. For the purposes of the disclosure herein, the term "AAV" is an abbreviation for the adeno-associated virus, including but not limited to the virus *per se* and derivatives thereof. Unless otherwise indicated, the term refers to all AAV subtypes or serotypes, and both replication-competent and recombinant forms.

The term "AAV" includes but is not limited to AAV1, AAV2, AAV 3A, AAV 3B, AAV 4, AAV 5, AAV 6, AAV 7, AAV 8, and AAV 9 types. The term "AAV" also includes AAVs with different origins, such as avian AAV, bovine AAV, canine AAV, caprine AAV, equine AAV, primate AAV, non-primate AAV, and ovine AAV. The term "primate AAV" refers to AAV that infects primates, "non-primate AAV" refers to AAV that infects non-primate mammals, etc.

The genomic sequences of various serotypes of AAV, as well as the sequences of native terminal repeats (ITRs), Rep proteins, and capsid subunits are known in the art. Such sequences can be found in the publications or in public databases such as GenBank.

The term "rAAV" refers to "recombinant AAV". In some embodiments, the recombinant AAV has an AAV genome in which part or all of the rep and cap genes have been replaced by heterologous sequences. If an AAV viral particle contains a heterologous polynucleotide (i.e., a polynucleotide other than the wild-type AAV genome), it is often referred to as a "recombinant AAV (rAAV) viral particle" or "rAAV viral particle". In general, the heterologous polynucleotide is flanked by at least one, and usually two, AAV inverted terminal repeats (ITRs).

The term "cap gene" or "capsid gene" refers to the nucleic acid sequence coding a capsid protein that forms or facilitates the formation of the capsid or protein shell of the virus. In the case of AAV, the capsid protein may be VP1, VP2, or VP3.

The term "rep gene" refers to the nucleic acid sequence coding non-structural proteins (rep78, rep68, rep52 and rep40, etc.) required for viral replication and production.

As used herein, the term "variant" refers to the polynucleotide or polypeptide having a sequence substantially similar to a reference polynucleotide or polypeptide. In the case of a polynucleotide, a variant may have a deletion, substitution, addition of one or more nucleotides at the 5' end, 3' end and/or one or more internal sites compared to the reference polynucleotide. Sequence similarities and/or differences between a variant and a reference polynucleotide may be detected by using conventional techniques known in the art (e.g., polymerase chain reaction (PCR) and hybridization techniques).

Variant polynucleotides also include synthetically derived polynucleotides, such as those generated by using site-directed mutagenesis. In general, a variant of a polynucleotide (including but not limited to DNA or RNA) may have at least 80%, at least 85%, at least 90%, at least 95%, at least 96%, at least 97%, at least 98% or at least 99% sequence identity with the reference polynucleotide as determined by sequence alignment programs known to those skilled in the art.

For a polypeptide or protein sequence, a variant may have a deletion, substitution, addition of one or more amino acids compared to the reference polypeptide or protein sequence. Sequence similarities and/or differences between variants and reference polypeptides can be detected by using conventional techniques known to those skilled in the art (e.g., Western blot). In general, a variant of a polypeptide may have at least 80%, at least 85%, at least 90%, at least 95%, at least 96%, at least 97%, at least 98%, or at least 99% sequence identity to the reference polypeptide as determined by sequence alignment programs known to those skilled in the art. In some cases, the term "variant" may be used to describe an AAV comprising a variant capsid sequence.

The term "operably linked" refers to two or more components (such as sequence elements) that are arranged so that both components function normally and at least one of the components is able to mediate a function imposed on at least one of the other components. For example, a transcriptional regulatory sequence such as a promoter is operably linked to a coding sequence if it controls the level of transcription of the coding sequence in response to the presence or absence of one or more transcriptional regulatory factors. A transcriptional regulatory sequence is generally operably linked to a coding sequence in cis, but is not necessarily directly adjacent to it. For example, an enhancer is a transcriptional regulatory sequence that is operably linked to a coding sequence, although they are not adjacent.

A polynucleotide "vector" or "construct" is capable of transferring a gene sequence to a target cell. In general, the terms "vector construct", "expression vector", "expression construct", "expression cassette", and "gene transfer vector" mean any nucleic acid construct that is capable of directing the expression of a gene of interest and transferring the gene sequence to a target cell. Thus, the term includes cloning and expression vectors as well as integration vectors.

The term "infectious" virus or viral particle indicates a virus or viral particle that contains a viral capsid that is capable of assembly and is capable of delivering polynucleotide components to a cell to which the viral species has tropism. The term does not necessarily imply any replication capability of the virus. Assays for counting infectious viral particles are described elsewhere in the present disclosure and in the art. Viral infectivity can be expressed as a ratio of infectious viral particles to total viral particles. Methods for determining the ratio of infectious viral particles to total viral particles are known in the art. See, e.g., Grainger et al. (2005) Mol. Ther. 11: S337 (describing TCID50 infection titer analysis); and Zolotukhin et al. (1999) Gene Ther. 6: 973.

The insect cells used in the production methods disclosed herein comprise a baculovirus helper construct having an expression cassette coding the modified cap gene described herein. The insect cells may be, but are not limited to, cultured cell lines such as BTI-TN-5B1-4 derived from Trichoplusia ni (High FiveTM, ThermoFisher Scientific, Carlsbad, CA), Sf9 cells or Sf21 cells (both of which are derived from Spodopterafrugiperda), Sf9 or TN368 cells with a mammalian-type glycan profile (coBac), or Sf-RVN cells (MilliporeSigma).

The insect cell may additionally comprise (on the same helper construct or on separate helper constructs) an expression cassette containing the AAV rep gene. The helper construct in the insect cell contains transcriptional regulatory elements that direct and regulate the expression of the Rep protein and the capsid protein. These elements include, but are not limited to, the constitutive or inducible promoters active in insect cells (e.g., p5 promoter, p10 promoter, p19 promoter, p40 promoter, polh promoter, E1 promoter, and ΔE1 promoter); Kozak sequence; transcription initiation and termination sites (modified or unmodified); mRNA splice sites (modified or unmodified, within or adjacent to the polypeptide coding sequence); and viral, eukaryotic or prokaryotic RNA elements that control splicing, nuclear export, localization, stability, or translation of a mRNA (e.g., woodchuck hepatitis virus posttranscriptional regulatory element (WPRE) (Zufferey et al., J Virol. 73(4):2886-92 (1999)), the MMLV/MPMV, eukaryotic constitutive transport element (CTE) (Li et al., Nature 443(7108):234-7 (2006)), RNA zipcode (Jambhekar and DeRisi, RNA 13(5):625-42(2007)), and omega or other 5'-UTR RNA elements that increase translation efficiency).

rAAV can contain an AAV vector containing a transgene of interest within its capsid. The transgene may encode a reporter protein for detection using biochemical (luciferase, SEAP) or for imaging (GFP, Venus, dTomato) techniques. The transgene may encode a therapeutic protein, which includes but is not limited to a chimeric antigen receptor (CAR), C-peptide or insulin, collagen VII, IGF-I, lipoprotein lipase, fibrinogen, prothrombin, factor V, factor VIII, factor IX, factor XI, factor XII, factor XIII, von Willebrand factor, prekallikrein, high molecular weight kininogen (Fitzgerald factor), fibronectin, antithrombin III, heparin cofactor II, protein C, protein S, protein Z, protein Z-related protease inhibitor, plasminogen, alpha 2-antiplasmin, tissue plasminogen activator, kinase, plasminogen activator inhibitor-1, and plasminogen activator inhibitor-2. The transgene may encode a sequence-specific nuclease (ZFN, TALEN or Cas9) or a sequence-specific binding protein (ZFP, TALE or dCas9). The transgene may encode an immunogenic protein for vaccination (e.g., a tumor antigen).

AAV vectors may contain transcriptional regulatory elements (e.g., promoters and enhancers) that can direct and regulate the expression of transgenes in human cells. AAV vectors may also contain the AAV complete or partial inverted terminal repeats (ITRs) at one or both ends of the transgene expression cassette. ITRs are required for packaging and viral integration into the host (human) genome.

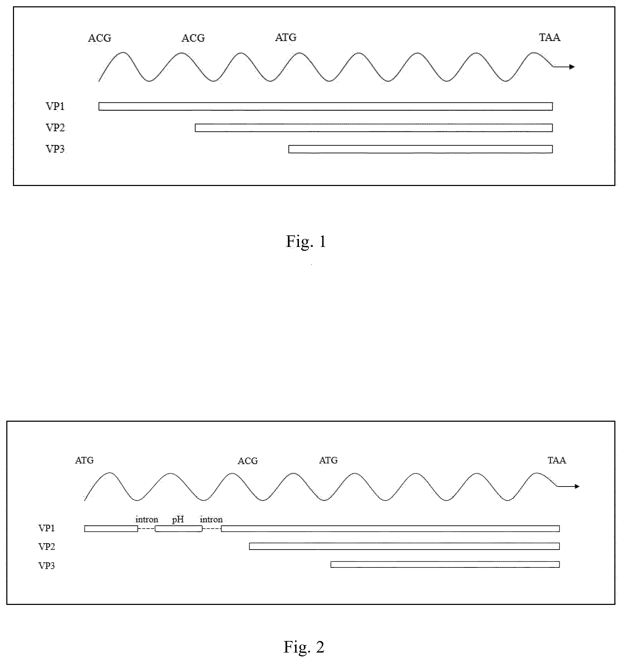

It is currently known that in mammalian cells, the optimal ratio for the capsid protein expression of wild-type AAV is approximately VP1:VP2:VP3=1:1:10. AAV capsid proteins expressed at this ratio are able to be efficiently assembled into structurally intact AAV particles. To this end, there are two main strategies for optimizing the capsid protein coding sequence in insect cells. One is to use a weakened start codon for VP1 (see Fig. 1), and the other is to add an intron sequence containing an insect cell promoter into the VP1-specific sequence (see Fig. 2).

The strategy using a weakened VP1 start codon achieves the simultaneous expression of the three types of VP proteins of AAV from a single gene sequence. However, since the start codon is weakened, other codons with stronger translation initiation functions in the translation expression frame will start translation at the same time. This competition will reduce the expression of complete VP1 and increase the expression of some incomplete (partial N-terminal sequence missing) VP1. These incomplete VP1 proteins participate in the assembly of AAV, which will eventually reduce the proportion of complete VP1 proteins in the packaged recombinant AAV capsid, thereby affecting infectivity.

Although the second method as shown in Fig. 2 weakens this effect, it is difficult to reach the wild-type VP1 ratio level, and the addition of an additional promoter will limit the subsequent combination optimization of recombinant vector elements.

The present disclosure refers to the usage frequency of promoters and weak promoter codons in insect cells, and performs synonymous mutations on weak promoter codons downstream of the primary promoter sequence in the VP1 of AAV capsid gene in insect cells to reduce the initial translation strength of these original codons, thereby reducing the expression of potentially incomplete VP1 and significantly improving the proportion of VP1 protein in AAV capsid proteins.

The present disclosure is further described in detail below in conjunction with the accompanying drawings and Examples. The following Examples are only used to illustrate the present disclosure and are not intended to limit the scope of the present disclosure. The experimental methods in the Examples that do not specify specific conditions are operated according to conventional conditions known in the art or according to the conditions recommended by the manufacturer.

### EXAMPLES

### Example 1. Construction of AAV8 and AAV6.2FF-related recombinant baculovirus shuttle vectors and obtainment of recombinant baculovirus

In this example, AAV8 and AAV6.2FF-related recombinant baculovirus shuttle vectors and recombinant baculovirus were obtained by the following steps.

Determination of related genes: The gene encoding the Rep protein was derived from AAV2 genome (GenBank: AF043303.1). Point mutations were introduced to the bases based on the ribosome leakage scanning mechanism, as described in the reference (Smith R H, Levy J R, Kotin R M. A simplified baculovirus-AAV expression vector system coupled with one-step affinity purification yields high-titer rAAV stocks from insect cells[J]. Molecular Therapy, 2009, 17(11): 1888-1896).

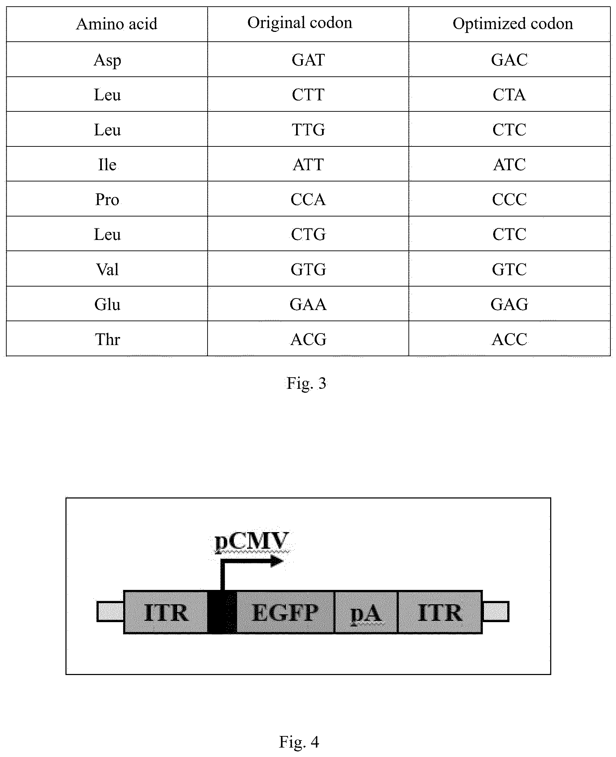

The gene encoding the Cap protein was derived from the AAV8 (NC_006261.1) and AAV6.2FF genome(van Lieshout L P, Domm J M, Rindler T N, et al. A novel triple-mutant AAV6 capsid induces rapid and potent transgene expression in the muscle and respiratory tract of mice[J]. Molecular Therapy-Methods & Clinical Development, 2018, 9: 323-329.). The optimization strategy is set forth in Fig. 3. The sequence of Acie01 can be found in GenBank: NC_001623.1, and the sequence of baculovirus homologous region hr1 can be found in GenBank: M14313.1.

GenScript and General Biosystems (Anhui) Co., Ltd. were commissioned to synthesize the corresponding sequences respectively: Rep gene sequence (nucleotide sequence as shown in SEQ ID NO:1), original AAV8 Cap gene sequence (nucleotide sequence as shown in SEQ ID NO:2), optimized AAV8 Cap VP1 gene sequence (nucleotide sequence as shown in SEQ ID NO:3), original AAV6.2FF Cap gene sequence (nucleotide sequence as shown in SEQ ID NO:4), optimized AAV6.2FF Cap VP1 gene sequence (nucleotide sequence as shown in SEQ ID NO:5), the combination of hr1 and p6.9 promoter, p10 promoter combination (hrlp6.9p10, nucleotide sequence as shown in SEQ ID NO:6), and the combination of pH promoter and Acie01 (pH-Acie01, nucleotide sequence as shown in SEQ ID NO:7).

Specifically, relative to the original AAV8 Cap gene sequence shown in SEQ ID NO:2, the codons in the VP1-specific region in SEQ ID NO:3 were optimized as follows: the codon GAT coding aspartic acid (Asp) was mutated to GAC; the codon CTT coding leucine (Leu) was mutated to CTA; the codon CCA coding proline (Pro) was mutated to CCC; the codon CTG coding leucine (Leu) was mutated to CTC; the codon GTG coding valine (Val) was mutated to GTC; the codon GAA coding glutamate (Glu) was mutated to GAG; and the codon ACG coding threonine (Thr) was mutated to ACC.

Relative to the original AAV6 Cap gene sequence set forth in SEQ ID NO:4, the VP1-specific region in SEQ ID NO:5 were optimized as follows: the codon GAT coding aspartic acid (Asp) was mutated to GAC; the codon ATT coding isoleucine (Ile) was mutated to ATC; the codon TTG coding leucine (Leu) was mutated to CTC; the codon CTG coding leucine (Leu) was mutated to CTC; the codon GTG coding valine (Val) was mutated to GTC; and the codon ACG coding threonine (Thr) was mutated to ACC.

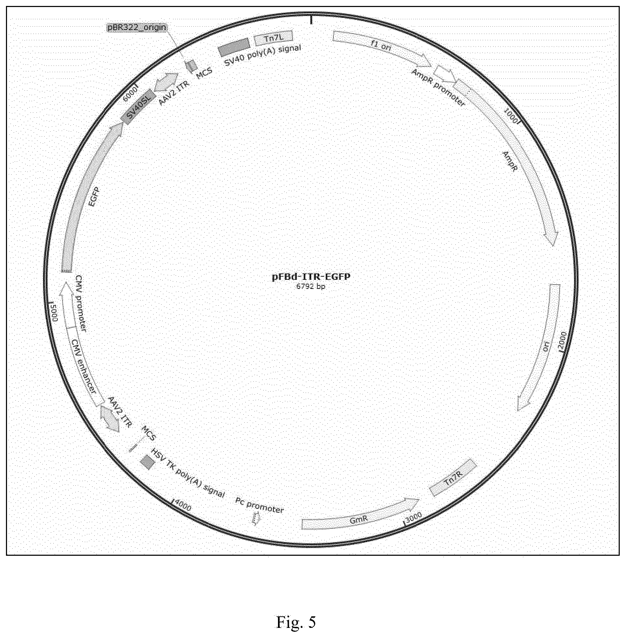

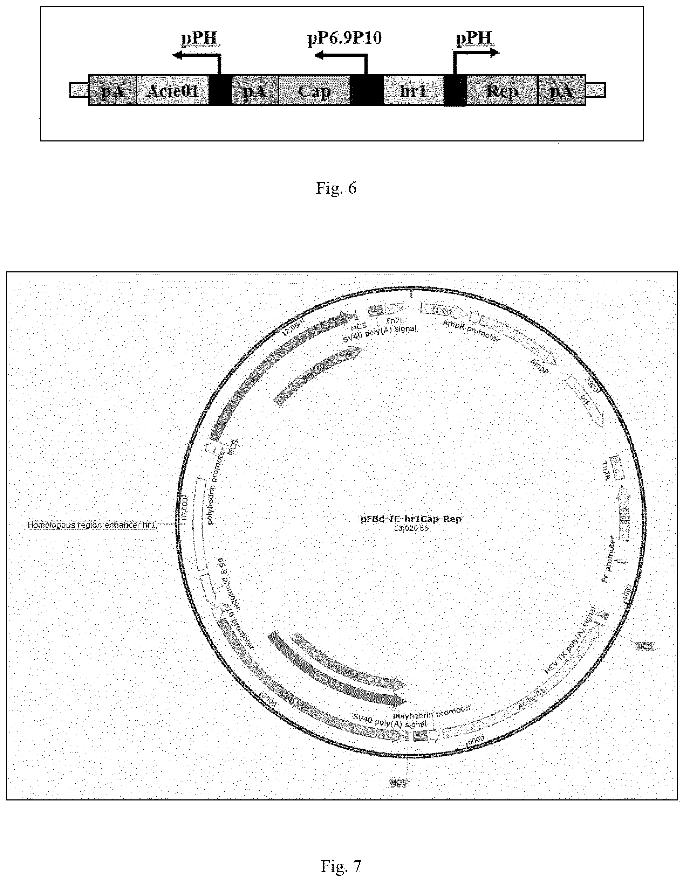

The ITR-CMV-EGFP sequence in the pAAV-EGFP vector (which contains the pUC57 backbone, upstream and downstream ITRs and the intermediate promoter CMV, the target gene EGFP, and the SV40 polyA) was cloned into the pFastBacdual vector (Invitrogen) using a homologous recombination method well known in the art. After sequencing identification, the recombinant baculovirus shuttle vector pFBd-ITR-EGFP was obtained (as shown in Figs. 4 and 5). The nucleotide sequence of the pFBd-ITR-EGFP vector is set forth in SEQ ID NO:8. Subsequently, the four Cap sequences were cloned into pFastBacdual, respectively, and after sequencing identification, four recombinant baculovirus shuttle vectors, namely, recombinant baculovirus shuttle vector pFBd-IE-hr1Cap8-Rep (nucleotide sequence shown in SEQ ID NO:9), pFBd-IE-hr1Cap8VP1Opti-Rep (nucleotide sequence shown in SEQ ID NO:10), pFBd-IE-hr1Cap6.2FF-Rep (nucleotide sequence shown in SEQ ID NO:11), and pFBd-IE-hr1Cap6.2FFVP1Opti-Rep (nucleotide sequence shown in SEQ ID NO:12) were obtained. The recombinant sequence and the recombinant baculovirus shuttle vector pFBd-IE-hr1Cap-Rep are shown in Figs. 6 and 7, respectively.

The recombinant vectors pFBd-ITR-EGFP, pFBd-IE-hr1Cap8-Rep, pFBd-IE-hr1Cap8VP1Opti-Rep, pFBd-IE-hr1Cap6.2FF-Rep and pFBd-IE-hr1Cap6.2FFVP1Opti-Rep were transformed into DH10Bac competent cells (Invitrogen) for blue-white screening. The white positive clones were selected for culture. Baculovirus plasmids (Bacmid) were extracted using PureLink^{™} HiPure Plasmid DNA Purification Kits (Invitrogen). Five Bacmids were separately transfected into adherent Sf9 cells (Gbico). After 4 days, the first-generation recombinant baculoviruses were harvested and amplified to the P2 generation. The P2 generation recombinant baculoviruses were named BV-ITR-EGFP, BV-IE-hr1Cap8-Rep, BV-IE-hr1Cap8VP1Opti-Rep, BV-IE-hr1Cap6.2FF-Rep and BV-IE-hr1Cap6.2FFVP1Opti-Rep, respectively.

The virus titers were determined by plaque assay, with specific reference to the Bac to Bac Expression System Operation Manual (Invitrogen).

### Example 2. Preparation of AAV-related recombinant baculovirus

The packaging and obtainment method of AAV is as follows: The P2-generation recombinant baculoviruses were used to co-infect 25 mL of suspended Sf9 cells (density 5.0E+06 cells/mL) at an MOI of 5.. The cells were harvested 4 days after infection and centrifuged at 4200 rpm for 10 minutes to separate the cells and culture supernatant. The culture supernatant was stored at 4 °C. The cells were lysed by incubating with 25 mL TNT lysis buffer (20 mM Tris-HCl (pH 7.5), 150 mM NaCl, 1% Triton X-100, 10 mmol/l MgCl₂) at room temperature for 1 hour. Nuclease was added at a final concentration of 100 U/mL, digesting overnight at 4 °C. The mixture was centrifuged at 10,000 rpm the next day. Finally, the supernatant after centrifugation was mixed with the cell culture supernatant to obtain a preliminary AAV virus stock solution.

Specifically, AAV8-EGFP was obtained by co-infecting Sf9 cells with BV-ITR-EGFP and BV-IE-hr1Cap8-Rep using the method described above; AAV8VP1Opti-EGFP was obtained by co-infecting Sf9 cells with BV-ITR-EGFP and BV-IE-hr1Cap8VP1Opti-Rep using the method described above; AA V6.2FF-EGFP was obtained by co-infecting Sf9 cells with BV-ITR-EGFP and BV-IE-hr1Cap6.2FF-Rep using the method described above; AAV6.2FFVP1Opti-EGFP was obtained by co-infecting Sf9 cells with BV-ITR-EGFP and BV-IE-hr1Cap6.2FF-Rep using the method described above.

### Example 3. Comparison of titers and infectivity of AAV-related recombinant baculoviruses

The physical titer of AAV8-EGFP, AAV8VP1Opti-EGFP, AAV6.2FF-EGFP and AAV6.2FFVP1Opti-EGFP virus stock solutions obtained in Example 2 was determined using a qPCR method well known in the art, and converted into the virus production titer per milliliter of cell culture volume. The final titer of AAV8-EGFP stock solution was 2.54×10¹² VG/mL, the titer of AAV8VP1Opti-EGFP stock solution was 8.53×10¹¹ VG/mL, the titer of AA V6.2FF-EGFP stock solution was 1.30×10¹² VG/mL, and the titer of AAV6.2FFVP1Opti-EGFP stock solution was 7.26×10¹¹ VG/mL.

The four virus stock solutions were purified using POROS^{™} CaptureSelect^{™} affinity chromatography filler (Thermo). The physical titer of the purified samples was determined using the same qPCR method. The purified samples were serially diluted and infected adherent 293T cells (obtained from ATCC, 48-well plate, 5.0 E+04 cells/well) at different MOIs. Two days after infection, the expression efficiency of EGFP was detected by flow cytometry.

The results showed that although the physical titers of AAV8VP1Opti-EGFP and AAV6.2FFVP1Opti-EGFP prepared after capsid optimization were about 3 times and 2 times lower than those of AAV8-EGFP and AAV6.2FF-EGFP with the original capsid, their biological infection performance was improved by 3.5 times and 5.3 times, respectively (Table 1). Therefore, after using this method to optimize the capsid, the yield of infectious AAV virus particles per unit cell culture volume was significantly improved.

**Table 1. Infectivity of AAV recombinant virus particles**

| GFP+ cell ratio | AAV8-WT | AAV8-opt |
|---|---|---|
| MOI=6400 | 4.82% | 16.68% |
| | 4.54% | 17.00% |
| MOI=12800 | 8.46% | 30.24% |
| | 8.00% | 29.64% |
| MOI=25600 | 14.52% | 47.13% |
| | 15.65% | 47.47% |

| GFP+ cell ratio | AAV6.2FF-WT | AAV6.2FF-opt |
|---|---|---|
| MOI=400 | 2.44% | 12.43% |
| | 2.12% | 12.89% |
| MOI=800 | 4.58% | 24.02% |
| | 4.17% | 22.16% |
| MOI=1600 | 8.81% | 40.86% |
| | 7.37% | 41.56% |

### Example 4. Capsid protein ratio of AAV-related recombinant baculovirus before and after optimization

Equal amounts (about 2×10¹¹ VG) of four AAV, i.e., AAV8-EGFP, AAV8VP1Opti-EGFP, AAV6.2FF-EGFP and AAV6.2FFVP1Opti-EGFP were subjected to SDS-PAGE using 0-20% precast gel (Nanjing GenScript Biotechnology Co., Ltd.). The specific steps were as follows: diluting the AAV samples with PBS to achieve a volume of 40 µL containing approximately 2×10¹¹ VG of AAV particles, adding 10 µL of 5x concentration loading buffer. The resulting mixture was mixed well and placed in a 100 °C metal bath for heating for 10 min. The SDS-PAGE instrument was assembled for sample loading. 40 µL of heated sample was loaded into each precast gel well. The protein marker (Thermofisher, 26616) was loaded in an amount of 8 µL. After sample loading, the SDS-PAGE apparatus was covered and connected to the electrophoresis apparatus. The voltage was set to 150 V. The electrophoresis apparatus was stopped once the bromophenol blue indicator migrated to 1-2 cm from the front edge. The total electrophoresis time was about 70 min.

After the electrophoresis was finished, the gel plate was taken out and placed in a container and stained overnight with 0.25% Coomassie brilliant blue dye. The next day, the staining solution was discarded. The gel surface was rinsed with distilled water several times and then the destaining solution was added for diffusion destaining. The destaining solution was changed every 30 min until the protein band became clear. The capsid ratio was analyzed.

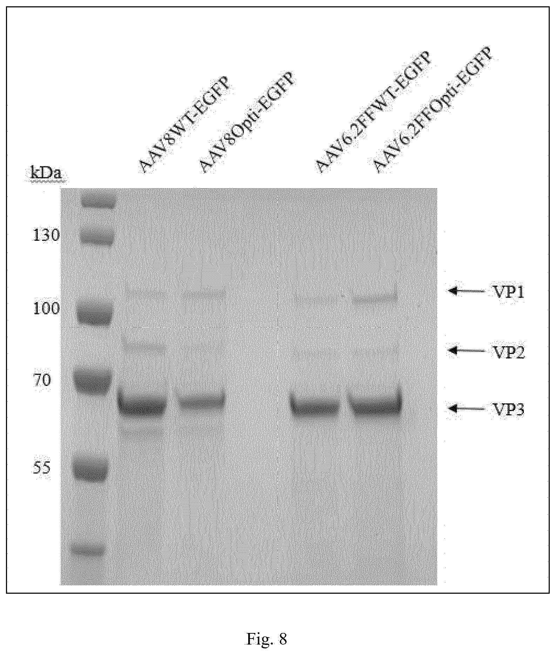

As shown in Fig. 8, the results showed that the proportion of VP1 in the virus with optimized capsid is significantly increased compared with the virus with the original capsid.

### Example 5. Construction of AAV9-related recombinant baculovirus shuttle vectors and obtainment and preparation of recombinant baculovirus

The gene of Cap protein was derived from the wild-type AAV9 VP1 gene (GenBank: AY530579). GenScript and General Biosystems (Anhui) Co., Ltd. were commissioned to synthesize the AAV9 cap VP1 gene sequence with the start codon changed to ACG (shown in SEQ ID NO:22), as well as the sequence optimized in the VP1-specific region of the Cap9 VP1opti-1 (shown in SEQ ID NO:23).

Relative to the sequence shown in SEQ ID NO:22 in which the AAV9 Cap VP1-specific region is not optimized, the codons in the VP1-specific region in VP1opti-1 sequence shown in SEQ ID NO:23 were optimized as follows: the codon GAT coding aspartic acid (Asp) was mutated to GAC (the mutation was to eliminate an abnormally encoded ATG after the start codon); the codon TTG coding leucine (Leu) was mutated to TTA; the codon GTG coding valine (Val) was mutated to GTC; the codon ACG coding threonine (Thr) was mutated to ACC; the codon CTG coding leucine (Leu) was mutated to CTC.

AAV9 Cap VP1 (shown in SEQ ID NO:22) was replaced on pFBd-IE-hr1Cap8-Rep by a homologous recombination method well known in the art. The recombinant baculovirus donor plasmid vector pFBd-IE-hr1Cap9-Rep was obtained after sequencing and identification. The optimized VP1 specific region Cap9 VP1opti-1 (shown in SEQ ID NO:23) was replaced on pFBd-IE-hr1Cap9-Rep by the homologous recombination method. The recombinant baculovirus donor plasmid vector pFBd-IE-hr1Cap9VP1Opti-1-Rep was obtained after sequencing and identification.

Using the site-directed mutagenesis and homologous recombination methods well known in the art, the 25th amino acid leucine codon in the VP1-specific region Cap9 VP1opti-1 of pFBd-IE-hr1Cap9VP1Opti-1-Rep was mutated into the weak synonymous codons CTA, CTC, and CTT, respectively. After mutation, they were named VP1opti-2 (shown in SEQ ID NO:24), VP1opti-3 (shown in SEQ ID NO:25), and VP1opti-4 (shown in SEQ ID NO:26), respectively. After sequencing and identification, the recombinant baculovirus donor plasmid vectors pFBd-IE-hr1Cap9VP1Opti-2-Rep, pFBd-IE-hr1Cap9VP1Opti-3-Rep, and pFBd-IE-hr1Cap9VP1Opti-4-Rep were obtained.

The optimization strategies in each vector are shown in the following table.

**Table 2. Optimization strategies in different vectors**

| Vector name | Characteristics in VP1 specific region |
|---|---|
| pFBd-IE-hr1Cap9-Rep | Wild type with ACG start codon |
| pFBd-IE-hr1Cap9VP1Opti-1-Rep | All of ACG (mutated to ACC), CTG (mutated to CTC), GTG (mutated to GTC), TTG contained therein were mutated to non-synonymous codons thereof, among which TTG was mutated to TTA; the potential ATG trinucleotide was mutated. |
| pFBd-IE-hr1Cap9VP 1 Opti-2-Rep | All of ACG (mutated to ACC), CTG (mutated to CTC), GTG (mutated to GTC), TTG contained therein were mutated to non-synonymous codons thereof, among which TTG was mutated to CTA; the potential ATG trinucleotide was mutated. |
| pFBd-IE-hr1Cap9VP 1 Opti-3-Rep | All of ACG (mutated to ACC), CTG (mutated to CTC), GTG (mutated to GTC), TTG contained therein were mutated to non-synonymous codons thereof, among which TTG was mutated to CTC; the potential ATG trinucleotide was mutated. |
| pFBd-IE-hr1Cap9VP 1 Opti-4-Rep | All of ACG (mutated to ACC), CTG (mutated to CTC), GTG (mutated to GTC), TTG contained therein were mutated to non-synonymous codons thereof, among which TTG was mutated to CTT; the potential ATG trinucleotide was mutated. |

The recombinant vectors pFBd-ITR-EGFP, pFBd-IE-hr1Cap9-Rep, pF pFBd-IE-hr1Cap9VP1Opti-1-Rep, pFBd-IE-hr1Cap9VP1Opti-2-Rep, pFBd-IE-hr1Cap9VP1Opti-3-Rep and pFBd-IE-hr1Cap9VP1Opti-4-Rep were transformed into DH10Bac competent cells (Invitrogen), respectively, for blue-white screening. The white positive clones were selected for culture. Baculovirus plasmids (Bacmid) were extracted using PureLink^{™} HiPure Plasmid DNA Purification Kits (Invitrogen). Five types of Bacmids were transfected into adherent Sf9 cells (Gbico), respectively. After 4 days, the first-generation recombinant baculovirus was harvested and amplified to the P2 generation. The obtained P2 generation recombinant baculoviruses were named BV-ITR-EGFP, BV-IE-hr1Cap9-Rep, BV-IE-hr1Cap9VP1Opti-1-Rep, BV-IE-hr1Cap9VP1Opti-2-Rep, BV-IE-hr1Cap9VP1Opti-3-Rep and BV-IE-hr1Cap9VP1Opti-4-Rep, respectively.

The virus titers were determined by plaque assay, with specific reference to the Bac to Bac Expression System Operation Manual (Invitrogen).

### Example 6: Preparation of AAV-related recombinant baculovirus

The packaging and production method of AAV is as follows: the P2 generation recombinant baculovirus was co-infected into 25 mL suspended Sf9 cells (at a density of 5.0E+06 cells/mL) at an MOI of 0.5. The cells were harvested 4 days after infection and centrifuged at 4200 rpm for 10 minutes to separate the cells and culture supernatant. The culture supernatant was stored at 4 °C. The culture supernatant was stored at 4 °C. The cells were lysed by incubating with 25 mL TNT lysis buffer (20 mM Tris-HCl (pH 7.5), 150 mM NaCl, 1% Triton X-100, 10 mmol/l MgCl₂) at room temperature for 1 hour. The nuclease was added at a final concentration of 100 U/mL. The mixture was centrifuged at 10,000 rpm. Finally, the supernatant after centrifugation was mixed with the cell culture supernatant to obtain a preliminary AAV virus stock solution.

Specifically, AAV9-WT-EGFP was obtained by co-infecting Sf9 cells with BV-ITR-EGFP and BV-IE-hr1Cap9-Rep using the method described above; AAV9VP1Opti-1-EGFP was obtained by co-infecting the Sf9 cells with BV-ITR-EGFP and BV-IE-hr1Cap9VP1Opti-1-Rep using the method described above; AAV9VP1Opti-2-EGFP was obtained by co-infecting the Sf9 cells with BV-ITR-EGFP and BV-IE- hr1Cap9VP1Opti-2-Rep using the method described above; AAV9VP1Opti-3-EGFP was obtained by co-infecting the Sf9 cells with BV-ITR-EGFP and BV-IE-hr1Cap9VP1Opti-3-Rep using the method described above; AAV9VP1Opti-4-EGFP was obtained by co-infecting the Sf9 cells with BV-ITR-EGFP and BV-IE-hr1Cap9VP1Opti-4-Rep using the method described above.

### Example 7: Comparison of titers and infectivity of AAV-related recombinant baculoviruses

The physical titer of the AAV9-WT-EGFP, AAV9VP1Opti-1-EGFP, AAV9VP1Opti-2-EGFP, AAV9VP1Opti-3-EGFP and AAV9VP1Opti-4-EGFP virus stock solutions obtained in Example 6 was determined using a qPCR method well known in the art, and converted into the virus production titer per milliliter of cell culture volume, as shown in Table 3 below.

**Table 3. Determination of AAV recombinant virus packaging yield**

| Sample name | Small-scale packaging yield VG/mL |
|---|---|
| AAV9-WT-EGFP | 1.08×10¹² |
| AAV9VP1Opti-1-EGFP | 1.05×10¹² |
| AAV9VP1Opti-2-EGFP | 1.17×10¹² |
| AAV9VP1Opti-3-EGFP | 1.08×10¹² |
| AAV9VP1Opti-4-EGFP | 1.11×10¹² |

The five types of virus stock solutions were purified using POROS^{™} CaptureSelect^{™} affinity chromatography filler (Thermo). The physical titer of the purified samples was determined using the same qPCR method. The purified samples were serially diluted and infected adherent 293T cells (obtained from ATCC, 48-well plate, 5.0 E+04 cells/well) at different MOIs. Two days after infection, the expression efficiency of EGFP was detected by flow cytometry.

**Table 4. Infectivity of AAV recombinant virus particles**

| Sample name | GFP+ cell ratio | | | |
|---|---|---|---|---|
| | MOI=1600 | MOI=3200 | MOI=6400 | MOI=12800 |
| AAV9-WT-EGFP | | 5.55% | 11.28% | 21.43% |
| AAV9VP1Opti-1-EGFP | 14.42% | 29.79% | 50.37% | 73.18% |
| AAV9VP1Opti-2-EGFP | 14.03% | 27.29% | 42.50% | 68.18% |
| AAV9VP1Opti-3-EGFP | 13.51% | 26.91% | 43.22% | 66.82% |
| AAV9VP1Opti-4-EGFP | 13.68% | 26.30% | 44.83% | 69.87% |

The experimental results showed that the AAV9VP1Opti-1-EGFP, AAV9VP1Opti-2-EGFP, AAV9VP1Opti-3-EGFP and AAV9VP1Opti-4-EGFP viruses prepared after capsid optimization had no significant difference in physical titer compared to the AAV9-EGFP with original capsid, but the biological infection performance was improved by more than 5 times (Table 4). Therefore, after using this method to optimize the capsid, the yield of infectious AAV virus particles per unit of cell culture volume was significantly improved.

### Example 8: Capsid protein ratio of AAV-related recombinant baculovirus before and after optimization

Equal amounts (about 2×10¹¹ VG) of AAV9-WT-EGFP, AAV9VP1Opti-1-EGFP, AAV9VP1Opti-2-EGFP, AAV9VP1Opti-3-EGFP and AAV9VP1Opti-4-EGFP were subjected to SDS-PAGE using 0-20% precast gel (Nanjing GenScript Biotechnology Co., Ltd.). The specific steps were as follows: diluting the AAV samples with PBS to achieve a volume of 40 µL containing approximately 2×10¹¹ VG of AAV particles, adding 10 µL of 5x concentration loading buffer. The resulting mixture was mixed well and placed in a 100 °C metal bath for heating for 10 min. The SDS-PAGE apparatus was assembled for sample loading. 40 µL of heated sample was loaded into each precast gel well. The protein marker (Thermofisher, 26616) was loaded in an amount of 8 µL. After sample loading, the SDS-PAGE apparatus was covered and was connected to the electrophoresis apparatus. The voltage was set to 150 V. The electrophoresis was stopped once the bromophenol blue indicator migrated to 1-2 cm from the front edge. The total electrophoresis time was about 70 min.

After the electrophoresis was finished, the gel plate was taken out and placed in a container and stained overnight with 0.25% Coomassie brilliant blue dye. The next day, the staining solution was discarded. The gel surface was rinsed with distilled water several times and then the destaining solution was added for diffusion destaining. The destaining solution was changed every 30 min until the protein band became clear. The capsid ratio was analyzed.

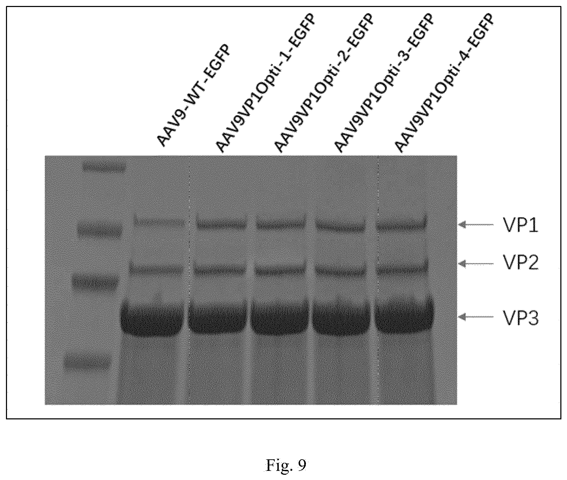

As shown in Fig. 9, the results showed that the proportion of VP1 in the virus with optimized capsid is significantly increased compared with the virus with the original capsid.

## Claims

1. A method for modifying the capsid protein coding sequence of the capsid protein coding gene (cap gene) of an adeno-associated virus (AAV), wherein the cap gene encodes capsid proteins VP1, VP2 and VP3 through one coding sequence with different start codons, the method comprising:
(1) using a weak start codon for the coding sequence of the VP1; and
(2) performing codon optimization for one or more codons in the coding sequence segment between the start codon of the coding sequence of the VP1 and the start codon of the coding sequence of the VP2 (VP1-specific segment), thereby reducing the initial translation strength of the codon.

2. The method according to claim 1, wherein:
(1) using the start codon ACG for the coding sequence of the VP1; and/or
(2) the codon optimization in the VP1-specific segment comprises: mutating all or part of the codons with stronger initiation function in the VP1-specific segment into synonymous codons except for the above codons.

3. A method for modifying the capsid protein coding sequence of the capsid protein coding gene (cap gene) of an adeno-associated virus (AAV), wherein the cap gene encodes capsid proteins VP1, VP2 and VP3 through one coding sequence with different start codons, the method comprising:
(1) using a weak start codon for the coding sequences of the VP1 and VP2 and a strong start codon for the coding sequence of the VP3; and
(2) performing codon optimization for one or more codons in the coding sequence segment between the start codon of the coding sequence of the VP1 and the start codon of the coding sequence of the VP2 (VP1-specific segment), thereby reducing the initial translation strength of the codon.

4. The method according to claim 3, wherein the start codon ACG is used for the coding sequences of the VP1 and VP2, and the start codon ATG is used for the coding sequence of VP3.

5. The method according to any one of claims 1 to 4, wherein in addition to the VP1-specific segment, one or more codons in additional coding sequence segment in the capsid protein coding sequence are codon-optimized to reduce the initial translation strength of the codon, and said additional coding sequence segment is selected from:
(1) a coding sequence segment between the start codon of the coding sequence of the VP2 and the start codon of the coding sequence of the VP3;
(2) a coding sequence segment downstream of the start codon of the coding sequence of the VP3; and
(3) a combination of (1) and (2).

6. The method according to any one of claims 1 to 5, wherein the codon optimization comprises one or more selected from the group consisting of:
(1) mutating the codon GAT coding aspartic acid (Asp) to a synonymous codon except for GAT, preferably to GAC, preferably mutating all GAT codons in the segment;
(2) mutating the codon CTT coding leucine (Leu) to a synonymous codon except for CTT, preferably to CTA, preferably mutating all CTT codons in the segment;
(3) mutating the codon TTG coding leucine (Leu) to a synonymous codon except for TTG, preferably to CTC, preferably mutating all TTG codons in the segment;
(4) mutating the codon ATT coding isoleucine (Ile) to a synonymous codon except for ATT, preferably to ATC, preferably mutating all ATT codons in the segment;
(5) mutating the codon CCA coding proline (Pro) to a synonymous codon except for CCA, preferably to CCC, preferably mutating all CCA codons in the segment;
(6) mutating the codon CTG coding leucine (Leu) to a synonymous codon except for CTG, preferably to CTC, preferably mutating all CTG codons in the segment;
(7) mutating the codon GTG coding valine (Val) to a synonymous codon except for GTG, preferably to GTC, preferably mutating all GTG codons in the segment;
(8) mutating the codon GAA coding glutamic acid (Glu) to a synonymous codon except for GAA, preferably to GAG, preferably mutating all GAA codons in the segment; and
(9) mutating the codon ACG coding threonine (Thr) to a synonymous codon except for ACG, preferably to ACC, preferably mutating all ACG codons in the segment.

7. The method according to claim 6, wherein the codon optimization comprises at least 2, at least 3, at least 4, at least 5, at least 6, at least 7, all 8 or all 9 of items (1) to (9).

8. The method according to claim 6 or 7, wherein the codon optimization comprises one or more selected from the group consisting of:
(1) mutating the codon TTG coding leucine (Leu) to a synonymous codon except for TTG, preferably to CTC, preferably mutating all TTG codons in the segment;
(2) mutating the codon CTG coding leucine (Leu) to a synonymous codon except for CTG, preferably to CTC, preferably mutating all CTG codons in the segment;
(3) mutating the codon GTG coding valine (Val) to a synonymous codon except for GTG, preferably to GTC, preferably mutating all GTG codons in the segment; and
(4) mutating the codon ACG coding threonine (Thr) to a synonymous codon except for ACG, preferably to ACC, preferably mutating all ACG codons in the segment.

9. The method according to claim 8, wherein the codon optimization comprises at least 2, at least 3 or all 4 of items (1) to (4).

10. The method according to any one of claims 1 to 9, wherein the method further comprises:
after the codon optimization, a trinucleotide ATG present in two adjacent codons in the capsid protein coding sequence is optimized, and the trinucleotide ATG is mutated to a non-ATG sequence without changing the amino acid encoded by the two adjacent codons.

11. The method according to any one of claims 1 to 10, wherein the AAV is of a serotype selected from the group consisting of AAV1, AAV2, AAV3, AAV4, AAV5, AAV6, AAV7, AAV8 and AAV9, preferably the AAV is of a serotype of AAV6, AAV8 or AAV9.

12. The method according to claim 11, wherein the AAV is of AAV6 serotype, and the codon optimization comprises the following codon optimization of the codons in the VP1-specific segment:
(1) mutating the codon GAT coding aspartic acid (Asp) to a synonymous codon except for GAT, preferably to GAC, preferably mutating all GAT codons in the segment;
(2) mutating the codon ATT coding isoleucine (Ile) to a synonymous codon except for ATT, preferably to ATC, preferably mutating all ATT codons in the segment;
(3) mutating the codon TTG coding leucine (Leu) to a synonymous codon except for TTG, preferably to CTC, preferably mutating all TTG codons in the segment;
(4) mutating the codon CTG coding leucine (Leu) to a synonymous codon except for CTG, preferably to CTC, preferably mutating all CTG codons in the segment;
(5) mutating the codon GTG coding valine (Val) to a synonymous codon except for GTG, preferably to GTC, preferably mutating all GTG codons in the segment;
(6) mutating the codon ACG coding threonine (Thr) to a synonymous codon except for ACG, preferably to ACC, preferably mutating all ACG codons in the segment.

13. The method according to claim 11, wherein the AAV is of AAV8 serotype, and the codon optimization comprises the following codon optimization of the codons in the VP1-specific segment:
(1) mutating the codon GAT coding aspartic acid (Asp) to a synonymous codon except for GAT, preferably to GAC, preferably mutating all GAT codons in the segment;
(2) mutating the codon CTT coding leucine (Leu) to a synonymous codon except for CTT, preferably to CTA, preferably mutating all CTT codons in the segment;
(3) mutating the codon CCA coding proline (Pro) to a synonymous codon except for CCA, preferably to CCC, preferably mutating all CCA codons in the segment;
(4) mutating the codon CTG coding leucine (Leu) to a synonymous codon except for CTG, preferably to CTC, preferably mutating all CTG codons in the segment;
(5) mutating the codon GTG coding valine (Val) to a synonymous codon except for GTG, preferably to GTC, preferably mutating all GTG codons in the segment;
(6) mutating the codon GAA coding glutamic acid (Glu) to a synonymous codon except for GAA, preferably to GAG, preferably mutating all GAA codons in the segment;
(7) mutating the codon ACG coding threonine (Thr) to a synonymous codon except for ACG, preferably to ACC, preferably mutating all ACG codons in the segment.

14. The method according to claim 11, wherein the AAV is of AAV9 serotype, and the codon optimization comprises the following codon optimization of the codons in the VP1-specific segment:
(1) mutating the codon TTG coding leucine (Leu) to a synonymous codon except for TTG, preferably to TTA, CTA, CTC or CTT, preferably mutating all TTG codons in the segment;
(2) mutating the codon CTG coding leucine (Leu) to a synonymous codon except for CTG, preferably to CTC, preferably mutating all CTG codons in the segment;
(3) mutating the codon GTG coding valine (Val) to a synonymous codon except for GTG, preferably to GTC, preferably mutating all GTG codons in the segment;
(4) mutating the codon ACG coding threonine (Thr) to a synonymous codon except for ACG, preferably to ACC, preferably mutating all ACG codons in the segment.

15. The method according to claim 14, wherein the method further comprises:
after the codon optimization, a trinucleotide ATG present in two adjacent codons in the capsid protein coding sequence is optimized, and the trinucleotide ATG is mutated to a non-ATG sequence without changing the amino acid encoded by the two adjacent codons.

16. The capsid protein coding sequence of the cap gene of an AAV obtained by the method of any one of claims 1 to 15.

17. A capsid protein coding sequence of the cap gene of a recombinant AAV, which has a nucleotide sequence as shown in SEQ ID NO:3 or SEQ ID NO:5, or a variant nucleotide sequence having at least 80%, at least 85%, at least 90%, at least 95%, at least 98%, or at least 99% sequence identity with the sequence shown in SEQ ID NO:3 or SEQ ID NO:5.

18. A capsid protein coding sequence of the cap gene of a recombinant AAV, wherein the VP1 promoter and the VP1-specific segment in the coding sequence have a nucleotide sequence as shown in any one of SEQ ID NOs:23-26, or a variant nucleotide sequence having at least 80%, at least 85%, at least 90%, at least 95%, at least 98%, or at least 99% sequence identity with the sequence shown in any one of SEQ ID NOs:23-26.

19. A capsid protein coding sequence of the cap gene of a recombinant AAV, wherein the cap gene encodes capsid proteins VP1, VP2 and VP3 through one coding sequence with different start codons, wherein:
(1) the coding sequence of the VP1 has a weak start codon;
(2) one or more codons in the coding sequence segment between the start codon of the coding sequence of the VP1 and the start codon of the coding sequence of the VP2 (VP1-specific segment) are codon-optimized, thereby reducing the initial translation strength of the codon.

20. The capsid protein coding sequence according to claim 19, wherein:
(1) the coding sequence of the VP1 has a start codon ACG; and/or
(2) codon optimization in the VP1-specific segment comprises: mutating all or part of the codons with stronger initiation function in the VP1-specific segment into synonymous codons except for the above codons.

21. A capsid protein coding sequence of the cap gene of a recombinant AAV, wherein the cap gene encodes capsid proteins VP1, VP2 and VP3 through one coding sequence with different start codons, wherein:
(1) the coding sequences of the VP1 and VP2 have weak start codons and the coding sequence of the VP3 has a strong start codon;
(2) one or more codons in the coding sequence segment between the start codon of the coding sequence of the VP1 and the start codon of the coding sequence of the VP2 (VP1-specific segment) are codon-optimized, thereby reducing the initial translation strength of the codon.

22. The capsid protein coding sequence of the cap gene of a recombinant AAV according to claim 21, wherein the coding sequences of the VP1 and VP2 have a start codon ACG, and the coding sequence of the VP3 has a start codon ATG.

23. The capsid protein coding sequence of the cap gene of a recombinant AAV according to any one of claims 19 to 22, wherein in addition to the VP1-specific segment, one or more codons in additional coding sequence segment in the capsid protein coding sequence are also codon-optimized to reduce the initial translation strength of the codon, and said additional coding sequence segment is selected from:
(1) a coding sequence segment between the start codon of the coding sequence of the VP2 and the start codon of the coding sequence of the VP3;
(2) a coding sequence segment downstream of the start codon of the coding sequence of the VP3; and
(3) a combination of (1) and (2).

24. The capsid protein coding sequence of the cap gene of a recombinant AAV according to any one of claims 19 to 23, wherein the codon optimization comprises one or more selected from the group consisting of:
(1) mutating the codon GAT coding aspartic acid (Asp) to a synonymous codon except for GAT, preferably to GAC, preferably mutating all GAT codons in the segment;
(2) mutating the codon CTT coding leucine (Leu) to a synonymous codon except for CTT, preferably to CTA, preferably mutating all CTT codons in the segment;
(3) mutating the codon TTG coding leucine (Leu) to a synonymous codon except for TTG, preferably to CTC, preferably mutating all TTG codons in the segment;
(4) mutating the codon ATT coding isoleucine (Ile) to a synonymous codon except for ATT, preferably to ATC, preferably mutating all ATT codons in the segment;
(5) mutating the codon CCA coding proline (Pro) to a synonymous codon except for CCA, preferably to CCC, preferably mutating all CCA codons in the segment;
(6) mutating the codon CTG coding leucine (Leu) to a synonymous codon except for CTG, preferably to CTC, preferably mutating all CTG codons in the segment;
(7) mutating the codon GTG coding valine (Val) to a synonymous codon except for GTG, preferably to GTC, preferably mutating all GTG codons in the segment;
(8) mutating the codon GAA coding glutamic acid (Glu) to a synonymous codon except for GAA, preferably to GAG, preferably mutating all GAA codons in the segment; and
(9) mutating the codon ACG coding threonine (Thr) to a synonymous codon except for ACG, preferably to ACC, preferably mutating all ACG codons in the segment.

25. The capsid protein coding sequence of the cap gene of a recombinant AAV according to claim 24, wherein the codon optimization comprises at least 2, at least 3, at least 4, at least 5, at least 6, at least 7, all 8 or all 9 of items (1) to (9).

26. The capsid protein coding sequence of the cap gene of a recombinant AAV according to claim 24 or 25, wherein the codon optimization comprises one or more selected from the group consisting of:
(1) mutating the codon TTG coding leucine (Leu) to a synonymous codon except for TTG, preferably to CTC, preferably mutating all TTG codons in the segment;
(2) mutating the codon CTG coding leucine (Leu) to a synonymous codon except for CTG, preferably to CTC, preferably mutating all CTG codons in the segment;
(3) mutating the codon GTG coding valine (Val) to a synonymous codon except for GTG, preferably to GTC, preferably mutating all GTG codons in the segment; and
(4) mutating the codon ACG coding threonine (Thr) to a synonymous codon except for ACG, preferably to ACC, preferably mutating all ACG codons in the segment.

27. The capsid protein coding sequence according to claim 26, wherein the codon optimization comprises at least 2, at least 3 or all 4 of items (1) to (4).

28. The capsid protein coding sequence according to any one of claims 19 to 27, wherein a trinucleotide ATG present in two adjacent codons in the capsid protein coding sequence is optimized, and the trinucleotide ATG is mutated into a non-ATG sequence without changing the amino acid encoded by the two adjacent codons.

29. The capsid protein coding sequence of the cap gene of a recombinant AAV according to any one of claims 19 to 28, wherein the AAV is of a serotype selected from the group consisting of: AAV1, AAV2, AAV3, AAV4, AAV5, AAV6, AAV7, AAV8 and AAV9, preferably the AAV is a serotype of AAV6, AAV8 or AAV9.

30. The capsid protein coding sequence according to claim 29, wherein the AAV is of AAV6 serotype, and the codon optimization comprises the following codon optimization of the codons in the VP1-specific segment:
(1) mutating the codon GAT coding aspartic acid (Asp) to a synonymous codon except for GAT, preferably to GAC, preferably mutating all GAT codons in the segment;
(2) mutating the codon ATT coding isoleucine (Ile) to a synonymous codon except for ATT, preferably to ATC, preferably mutating all ATT codons in the segment;
(3) mutating the codon TTG coding leucine (Leu) to a synonymous codon except for TTG, preferably to CTC, preferably mutating all TTG codons in the segment;
(4) mutating the codon CTG coding leucine (Leu) to a synonymous codon except for CTG, preferably to CTC, preferably mutating all CTG codons in the segment;
(5) mutating the codon GTG coding valine (Val) to a synonymous codon except for GTG, preferably to GTC, preferably mutating all GTG codons in the segment;
(6) mutating the codon ACG coding threonine (Thr) to a synonymous codon except for ACG, preferably to ACC, preferably mutating all ACG codons in the segment.

31. The capsid protein coding sequence according to claim 29, wherein the AAV is of AAV8 serotype, and the codon optimization comprises the following codon optimization of the codons in the VP1-specific segment:
(1) mutating the codon GAT coding aspartic acid (Asp) to a synonymous codon except for GAT, preferably to GAC, preferably mutating all GAT codons in the segment;
(2) mutating the codon CTT coding leucine (Leu) to a synonymous codon except for CTT, preferably to CTA, preferably mutating all the CTT codons in the segment;
(3) mutating the codon CCA coding proline (Pro) to a synonymous codon except for CCA, preferably to CCC, preferably mutating all CCA codons in the segment;
(4) mutating the codon CTG coding leucine (Leu) to a synonymous codon except for CTG, preferably to CTC, preferably mutating all CTG codons in the segment;
(5) mutating the codon GTG coding valine (Val) to a synonymous codon except for GTG, preferably to GTC, preferably mutating all GTG codons in the segment;
(6) mutating the codon GAA coding glutamic acid (Glu) to a synonymous codon except for GAA, preferably to GAG, preferably mutating all GAA codons in the segment;
(7) mutating the codon ACG coding threonine (Thr) to a synonymous codon except for ACG, preferably to ACC, preferably mutating all ACG codons in the segment.

32. The capsid protein coding sequence according to claim 29, wherein the AAV is of AAV9 serotype, and the codon optimization comprises the following codon optimization of the codons in the VP1-specific segment:
(1) mutating the codon TTG coding leucine (Leu) to a synonymous codon except for TTG, preferably to TTA, CTA, CTC or CTT, preferably mutating all TTG codons in the segment;
(2) mutating the codon CTG coding leucine (Leu) to a synonymous codon except for CTG, preferably to CTC, preferably mutating all CTG codons in the segment;
(3) mutating the codon GTG coding valine (Val) to a synonymous codon except for GTG, preferably to GTC, preferably mutating all GTG codons in the segment;
(4) mutating the codon ACG coding threonine (Thr) to a synonymous codon except for ACG, preferably to ACC, preferably mutating all ACG codons in the segment.

33. The capsid protein coding sequence according to claim 32, wherein a trinucleotide ATG present in two adjacent codons in the capsid protein coding sequence is optimized, and the trinucleotide ATG is mutated into a non-ATG sequence without changing the amino acid encoded by the two adjacent codons.

34. The capsid protein coding sequence of the cap gene of a recombinant AAV according to claim 19, which has a nucleotide sequence as shown in SEQ **ID** NO:3 or SEQ **ID** NO:5, or a variant nucleotide sequence having at least 80%, at least 85%, at least 90%, at least 95%, at least 98%, or at least 99% sequence identity with the sequence shown in SEQ **ID** NO:3 or SEQ ID NO:5.

35. The capsid protein coding sequence of the cap gene of a recombinant AAV according to claim 19, wherein the VP1 promoter and the VP1-specific segment in the coding sequence have a nucleotide sequence as shown in any one of SEQ ID NOs:23-26, or a variant nucleotide sequence having at least 80%, at least 85%, at least 90%, at least 95%, at least 98%, or at least 99% sequence identity with the sequence shown in any one of SEQ ID NOs:23-26.

36. A vector comprising the capsid protein coding sequence of the cap gene of a recombinant AAV according to any one of claims 19 to 35, preferably the vector is an expression vector.

37. The vector according to claim 36, wherein the vector is a viral vector, preferably an insect baculovirus vector.

38. The vector according to claim 37, wherein the vector is derived from a vector system selected from: Bac-to-Bac vector system, flashBac/BacMagic vector system, BaculoDirect vector system or BacPAK6/Bacologold vector system.

39. The vector according to any one of claims 36 to 38, wherein the vector further comprises a replication-associated protein gene (rep gene) of AAV, preferably the rep gene is derived from AAV2 serotype.

40. The vector according to any one of claims 36 to 39, wherein the vector further comprises a nucleotide sequence coding a protein of interest.

41. A cell, wherein the cell comprises the vector according to any one of claims 36 to 40, preferably the cell is an insect cell.

42. A method for preparing a recombinant AAV, the method comprising:
(1) culturing the cell according to claim 41; and
(2) isolating the recombinant AAV from the cell and/or the culture supernatant.

43. The method according to claim 42, wherein when expressed in the cell, the expression amount of VP1, VP2 and VP3 is in a ratio of about 1:1:10.
